# EUROPEAN PATENT APPLICATION

(11) **EP 0 896 002 A1**
(43) Date of publication of application: **10.02.1999**
(21) Application number: 98901043.4
(22) Date of filing: 29.01.1998
(51) Int. Cl.: C07K 14/705, C12N 15/12, G01N 33/50

(54) **CHIMERIC PROTEINS, HETERODIMER COMPLEXES THEREOF AND SUBSTITUTE FOR PLATELET**

(30) Priority: 29.01.1997 JP 15118/97; 29.08.1997 JP 234544/97
(71) Applicant: TORAY INDUSTRIES, INC., Tokyo 103 (JP)
(72) Inventor: KAINOH, Mie, Kanagawa 251 (JP); TANAKA, Toshiaki, kanagawa 249 (JP)
(74) Representative: Kador & Partner
(86) International application number: JP9800370
(87) International publication number: WO9832771

(57) **Abstract**

Integrin-immunoglobulin chimeric protein heterodimer complexes in which the α-chain and β-chain of integrins have been associated in a stable state. These complexes are not only usable as drugs as such but also applicable to the assaying of binding of integrins to ligands and the detection of substances binding to integrins or those inhibiting binding of integrins to ligands. These comnplexes are also usable as diagnostic agents. It has been found that integrins isolated in a stably associated structure would bind to extracellular matrixes under physiological conditions in the presence of plasma components, which indicates that integrins and, in its turn, extracellular matrix receptors might be usable as substitutes for platelets.

## Description

### Technical Field:

The present invention relates to chimeric proteins consisting of an integrin and an immunoglobulin, their heterodimer complexes, a production process thereof, their applications as drugs and reagents, etc. Furthermore, the present invention relates to medicinal application of isolated extracellular maxtrix receptors such as integrin-immunoglobulin chimeric protein heterodimer complexes, as platelet substitutes.

### Background Arts:

Various cells have receptors which mediate the adhesion between a cell and a cell and receptors which mediate the adhesion between a cell and an extracellular matrix, and these receptors play important roles in immune reaction, inflammatory reaction, development, morphogenesis, wound healing, hemostasis, cancerous metastasis, etc. By separating and identifying the receptors which participate in these phenomena, the existence of so-called cell adhesion molecules has been clarified. Many of the molecules identified one after another are classified in reference to their structural features into integrin superfamily, immunoglobulin superfamily, select in family, cadherin family, etc. (Corlos, T. M. and Harlan, J. M., Blood, 84, 2068-2101 (1994)). Of these families, the immunoglobulin superfamily, selectin family and cadherin family mediate mainly the adhesion between a cell and a cell, while the integrin superfamily is the so-called extracellular matrix receptors which mediate the adhesion to extracellular matrices such as fibronectin and collagens. In addition, extracellular matrix receptors which do not belong to any of these adhesion molecule families include CD26 (DDPIV), CD44, GPIV, GPVI, GPIb-vWF, etc. CD26 is a receptor for collagens, and CD44 is a receptor for hyaluronic acid, fibronectin and collagens ("Adhesion Molecules" p. 32-42, Masayuki Miyasaka (1991), Medical View (in Japanese)). Furthermore, it is reported that among the membrane glycoproteins (GPs) existing on platelets, GPIV, GPVI, GPIb-vWF, etc. are also collagen receptors ("Platelet Receptors", p. 119-132, Minoru Ohkuma et al., (1992), Kinpodo (in Japanese)).

A receptor belonging to the integrin superfamily has a heterodimer complex structure in which two subunits, α-chain and β-chain as mutually different membrane proteins are associated with each other non-covalently (Hynes, R. O., Cell, 48, 549-554 (1987)). In the past, the integrin superfamily was classified into three subfamilies; β1 integrin, β2 integrin and β3 integrin. Later, new β chains and α chains were discovered one after another, and presently eight β chains (β1, β2 β3, β4, β5, β6, β7 and β8 and fifteen α chains (α1, α2, α3, α4, α5, α6, α7, α8, α9, αv, αL, αM, αX, αIIb and αE) have been identified (Elner, S. G. and Elner, V. M. Inv. Ophtal. Vis. Sci., 37, 696-701 (1996)). It is known that each β chain is associated with one to eight α chains, and as a result, 21 pairs of an α chain and a β chain, i.e., integrin molecules have been identified (Elner, S. G. and Elner, V. M., Inv. Ophtal, Vis. Sci., 37, 696-701 (1996)). They include α 4β1 (VLA-4, β1 integrin), αLβ2 (LFA-1, β2 integrin), αMβ2 (Mac-1, β2 integrin), αIIbβ3 (GPIIb/IIIa, β3 integrin), etc. now targeted for drug development (Drug and Market Development, 6, 201-205 (1995)). Many other integrins are also expected to have relations with diseases.

The heterodimer complex structure of an integrin plays an important role in binding to a ligand (Hynes, R. O., Cell, 48, 549-554 (1987)). For example, it is estimated that the ligand binding region on an integrin consists of both an α chain and a β chain (Hynes, R. O., Cell, 69, 11-25 (1992)). The fact that integrins having the same α chain but associated with a different β chain, or integrins having the same β chain but associated with a different α chain are respectively different in substrate specificity (Elner, S. G. and Elner, V. M., Inv. Ophtal, Vis. Sci. 37, 696-701 (1996)) supports this assumption. On the other hand, it was reported that the α chains of some integrins have an sequence called an I domain consisting of about 180 amino acids inserted in the molecule, and data suggest that the I domain only could be bound to a ligand were reported (Ueda, T. et al., Proc. Natl. Acad. Sci. USA, 91, 10680-10684 (1994)). However, it was also reported that the I domain of an α domain and the integrin as its original heterodimer complex are different in the style of binding to a ligand (Kamata, T. and Takada, Y., J. Biol. Chem., 269, 26006-26010 (1994)). It is also not clarified yet whether such parameters as specificity and affinity to a ligand are identical. It is not reported that in the case of an integrin not containing the I domain, for example, in the case of α4β1 a partial structure only is bound to a ligand.

If any integrin isolated and prepared retains its heterodimer complex structure, hence the ligand binding capability, it can be used for studying the style of binding to a ligand in a state close to nature. Furthermore, it can be used as it is as a drug and can also be used as a reagent for measuring the amount of a ligand in tissue or serum or as a material for searching for adhesion inhibiting compounds very usefully. However, isolating and preparing an integrin with its function retained is said to be very difficult. One reason is that since the association between an α chain and a β chain of an integrin is maintained non-covalently as desribed before, they are easily dissociated during isolation and preparation. Since an integrin is a membrane protein, the necessity of using a surfactant, etc. for solubilization is considered to be a large cause in the dissociation of the complex. In other words, the non-covalent preservation of functional structure inhibits the preparation of such an integrin.

Inspite of the difficulty as described above, some cases were reported, in which an integrin heterodimer complex was isolated and prepared with its function retained. For cases of α2β1, α5β1 and αvβ3, it was reported that the binding to a ligand can be determined by letting a liposome incorporate an integrin purified by using affinity column chromatography (Santoro, S. A. et al., Biochem. Biophys. Res. Comm., 153, 217-223 (1988), Pytela, R. et al. Cell, 40, 191-198 (1985), Pytela, R. et al., Method Enzymol., 144, 475-489 (1987)). For other cases, it was that if purified α5β1 or αvβ3 is coated on a plate, a peptide which inhibits the cell adhesion through the integrin can be selected (Koivunen, E. et al., J. Biol. Chem., 268, 20205-20210 (1993), Healy, J. M. et al., Biochemistry, 34, 3948-3955 (1995)). For further other cases, it was reported that if purified αvβ3 or α4β1 is coated on a plate, the binding to a ligand can be determined (Charo, I. F. et al., J. Cell Biol., III, 2795-2800 (1990), Makarem, R. et al., J. Biol. Chem., 269, 4005-4011 (1994), Paul Mould, A. et al., J. Biol. Chem., 269, 27224-27230 (1994)). For a still further other case, it was reported that if an extracellar portion of αIIbβ3 heterodimer complex prepared by gene manipulation is coated on a plate through a complex specific antibody, the binding to a ligand can be determined (Gulino, D. et al., Eur. J. Biochem., 227, 108-115 (1995)). These cases suggest that to exert the function of a purified integrin, its heterodimer complex must be bound to or included in any carrier. The reason why a carrier is considered to be necessary is that since a heterodimeter complex is associated non-covalently in a solution, it tends to be dissociated and as a result, cannot retain its fuctional structure. In the finally stated case, only a molecule with a heterodimer complex structure is selected using a complex specific anti body, in a design to determine the binding in a state where both the chains are not dissociated from each other.

As a case requiring no carrier, it was reported that purified α1β1, or α2β1 allows the determination of the bonding to a ligand dependent on high concentration of metal ions even without using any carrier (Pfaff, M. et al., Eur. J. Biochem., 225, 975-984 (1994)). In this case, the surfactant added in the process of purification plays a role similar to that of a liposome, acting as a carrier. For a further other case, it was reported that an extracellar of αMβ2 heterodimer complex prepared by using gene manipulation is bound to a ligand (Berman, P. W. et al., J. Cell Biochem., 52, 183-195 (1993)). These cases do not suggest the necessity of any carrier as described before, but the disadvantage that the association of molecules in a heterodimer complex is retained non-covalently is not improved.

As a still further other case, a chimeric protein consisting of αd and an immunoglobulin is disclosed (Japanese Patent Laid-Open (Kokai) No. 8-507933), but only the result of immune precipitation is reported, without examining the binding to a ligand. Furthermore, since a β chain is not expressed in the chimeric protein as an immunoglobulin, the binding between an α chain and a β chain remains non-covalent.

The above facts suggest that any integrin with an α chain and a β chain structurally stably associated and with its function retained has never been successfully prepared. That a complex structure is unstable restricts the use of its molecule.

Of the molecules belonging to the integrin superfamily, integrin α2β1 is an extracellular matrix receptor found to be expressed in T cells, platelets, etc. activated for long time. However, it was reported that the α2β1 on the cell surfaces of platelets and fibroblasts is bound to collagens only and that the α2β1 on the surfaces of vascular endothelial cells is bound to both collagens and laminins (Elices, M. J. et al., Proc. Natl. Acad. Sci. USA, 86, 9906-9910 (1989)), and it is speculated that the function of α2β1 becomes different, depending on cells.

In relation to the conditions of diseases, there are reports to suggest that integrin α2β1 plays an important roll for wound healing and cancerous metastasis (Shiro, J. A. et al., Cell, 67, 403-410 (1991), Chen, F. et al., J. Exp. Med., 173, 1111-1119 (1991), Chan, B. M. C. et al., Science, 251, 1600-1602 (1991)). Furthermore, it was reported that from the analysis of platelet function of patients with bleeding tendency, the adherence of platelets and collagens through integrin α2β1 has close relation with the first step of hemostasis and thrombosis process (Nieuwenhuis, H. K. et al., Nature, 318, 470-472 (1985)). Though the relations of integrin α2β1 with conditions of diseases are suggested like this, any medical application of using the integrin α2β1 protein and other isolated extracellular matrix receptor proteins under physiological ion condition or in the presence of plasma components has not been examined.

On the other hand, the necessity for artificial substitutes of platelets used as blood preparations in the clinical field is growing, and various attempts have been reported (Progress of Medicine 179, 406-407 (1996), Clinical Blood 37, 1353-1361 (1997) (respectively in Japanese)). However, they are not yet practically available.

### Disclosure of the Invention:

The present invention relates to chimeric proteins in which the α chain and β chain of an integrin are combined with the heavy chain or light chain of an immunoglobulin, their heterodimer complexes, a production process thereof, a method for testing the binding of an integrin-immunoglobulin chimeric protein heterodimer complex to a ligand and a cell, substances bound to an integrin obtained by using the method, a method for searching for a substance inhibiting the binding between an integrin and a ligand using the integrin-immunogobulin chimeric protein heterodimer complex, substances for inhibiting the binding, and the application of integrin-immunoglobulin chimeric protein heterodimer complexes as drugs and reagents. Furthermore, the present invention relates to platelet substitutes containing an integrin-immunoglobulin chimeric protein heterodimer complex or any other isolated extracllular matrix receptor as an active ingredient.

### Brief Description of the Drawings:

Fig. 1 shows that α4·IgG heavy chain-β1·IgG heavy chain chimeric protein heterodimer complex is bound to VCAM-1 expressing cell, and that the binding is inhibited by an anti-integrin antibody or EDTA, a cationic cheating agent.
Fig. 2 shows that α4·IgG heavy chain-β1·IgG heavy chain chimeric protein heterodimer complex is bound to CS-1 peptide, and that the binding is Inhibited by an anti-integrin antibody or EDTA, a cationic cheating agent.
Fig. 3 shows that the binding between α4·IgG heavy chain-β1IgG heavy chain chimeric protein heterodimer complex and CS-1 piptide is inhibited by GPEILDVPST, and is not inhibited by any other peptide.
Fig. 4 shows that α2·IgG heavy chain-β1gG heavy chain chimeric protein heterodimer complex is bound to a collagen, and that the binding is inhibited by an anti-integrin antibody and EDTA, a cationic cheating agent.
Fig. 5 shows that α2·IgG heavy chain-β1IgG heavy chain chimeric protein heterodimer complex liposome is bound to a collagen in the presence of plasma.
Fig. 6 shows that the binding of α2·IgG heavy chain-β1IgG heavy chain chimeric protein heterodimer complex liposome to a collagen is inhibited by an anti-integrin antibody or EDTA, a cationic chelating agent.

### The Best Embodiments of the Invention:

The extracellular matrix receptors in the present invention refer generally to the receptors which mediate the adhesion between a cell and an extracellular matrix. The receptors include the integrin superfamily having a heterodimer complex structure in which an α chain and a β chain are non-covalently associated with each other as two membrane proteins (Corlos, T. M. and Harlan, J. M. Blood, 84, 2068-2101 (1994)), and other receptors such as CD26 (DDPIV), CD44, GPIV, GPVI, GPb-vWF, etc. The integrins in the present invention refer to molecules belonging to the integrin superfamily, and also include the isomers of the molecules belonging to the family. The α chains of the present invention include 15 α chains, i.e., α1, α2, α3, α4, α5, α6, α7, α8, α9, αv, αL, αM, αX, αIIb and αE, and among them, α4 and α2 are preferable, though preferable α chains are not limited to them. The β chains of the present invention include eight β chains, i.e., β1, β2, β3, β4, β5, β6, β7and β8 and among them, β1 is preferable, though preferable β chains are not limited to it. The integrin molecules as pairs respectively consisting of an α chain and a β chain include the twenty one integrins stated in Elner, S. G. and Elner, V. M., Inv. Ophtal. Vis. Sci., 37, 696-701 (1996), though not limited to them.

A chimeric protein consisting of the α chain of an integrin and the heavy chain or light chain of an immunoglobulin refers to a molecule in which the extracellular region of the α chain of an integrin is bound to the constant region of the heavy chain or light chain contained an immunoglobulin. In this case, a chimeric protein in which N terminus side of the protein is integrin molecule and then connected to an immunoglobulin molecule side by side is preferable. A chimeric protein consisting of the β chain of an integrin and the heavy chain or light chain of an immunoglobulin refers to a molecule in which the extracellular region of the β chain of an integrin is bound to the constant region of the heavy chain or light chain contained in an immunoglobulin. Also in this case, a chimeric protein in which N terminus side of the protein is an integrin molecule and then connected to an immunoglobulin molecule side by side is preferable. In either case of α chain or β chain, a chimeric protein bound to the heavy chain of an immunoglobulin is preferable.

The isotype of the immunoglobulin to be bound to the α chain or β chain is not especially limited. Any of IgG, IgM, IgA and IgE can be used, but it is preferable to use IgG. The subclasses of IgG include IgG₁, IgG₂, IgG₃ and IgG₄, but it is preferable to use IgG 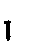 . Furthermore, it is possible to use a molecule with a diner structure having a disulfide bond between molecules instead of the immunoglobulin.

In the present invention, a molecule in which a chimeric protein consisting of the α chain of an integrin and the heavy chain or light chain of an immunoglobulin and a chimeric protein consisting of the β chain of the integrin and the havy chain or light chain of the immunoglobuline are associated with each other is called an integrin-immunoglobulin chimeric protein heterodimer complex. In this case, a combination consisting of α chain·immunoglobulin heavy chain (which means a chimeric protein consisting of an α chain and the heavy chain of an immunoglobulin; hereinafter this applies) and β chain·immunoglobulin heavy chain, a combination consisting of α chain·immunoglobulin heavy chain and β chain·immunoglobulin light chain, and a combination consisting of α chain·imminoglobulin light chain and β chain·immunoglobulin heavy chain are preferable. A combination consisting of α chain·immunoglobulin heavy chain and β chain·immunoglobulin heavy chain is more preferable.

In the integrin-immunoglobulin chimeric protein heterodimer complex of the present invention, the α chain can be α1, α2, α3, α4, α5, α6, α7, α8, α9, αv, αL, αM, αX, αIIb or αE, and the β chain can be β1, β2, β3, β4, β5, β6, β7 or β8, It is preferable that the α chain is α4 or α2 and that the β chain is β1, though preferable chains are not limited to them.

The process for preparing an integrin-immunogloburin chimeric protein heterodimer complex is described below, but the process is not limited thereto.

A DNA coding for the α chain and β chain of an integrin can be obtained using the information of known cDNA sequences by such a method as gene amplification based on the PCR method, cDNA cloning or DNA synthesis. For example, the DNA sequences of α4 and β1 are already reported in literature (Takada, Y. et al., EMBO J., 8, 1361-1368 (1989), Scott Argraves, W. et al., J. Cell Biol., 105, 1183-1190 (1987)). A DNA coding for the α chain and β chain of an integrin can also he obtained by the expression cloning using an antibody, etc. For binding to a DNA coding for the constant region of an immunoglobulin, it is desirable to take out a DNA coding for the extracellular portions only of the α chain and β chain of an integrin. For this purpose, it is preferable to use the PCR method and DNA synthesis. The extracellular portion of either an α chain or β chain refers to the polypeptide sequence on the N terminus side from the portion speculated to be the transmembrane portion. Its partial sequence can also be used as far as the ligand binding capability is retained, but it is preferable to use most of the portion considered to be an extracellular region. For taking out a DNA, it is necessary to adjust for adaptation of frames after linking to a DNA coding for an immunoglobulin. For example, this can be achieved by modifying the primer when a DNA fragment is taken out by the PCR method. In this case, it is desirable to design for ensuring that amino acid modification is not caused by the base substitution of the primer. However, amino acid substitution is allowed as far as the function of the chimeric protein is not changed. For obtaining a DNA by chemical synthesis, the purpose can be achieved by designing a sequence to ensure the linking to a DNA coding for an immunoglobulin. In the case of cDNA, a DNA capable of being bound to a DNA coding for an immunoglobulin can be prepared by using DNA fragmentation and a synthetic DNA.

Then, a DNA coding for an immunoglobulin is prepared. In the present invention, it is desirable to use DNAs coding for the heavy chain and light chain of a human immunoglobulin, but DNAs coding for an immunoglobulin of another animal species can also be used. The preparation of a DNA coding for human IgG is already reported (Ellison, J. W. et al., Nucleic Acids Res., 10, 4071-4079 (1982)), but the preparation is not limited to this method. Any method similar to the above mentioned method for preparing DNAs coding for the α chain and β chain of an integrin can also be used. In the present invention, for the heavy chain of a human immunoglobulin, it is preferable to use a genomio DNA, but a cDNA can also be used. As the DNA for the heavy chain of a human immunoglobulin, it is preferable to use a portion coding for the hinge region, CH2 region or CH3 region, but a DNA coding for the entire constant region of CH1 ⁻ CH3 can also be used. For the light chain of an immunoglobulin, a DNA coding for the CL region is used. Finally, a DNA coding for the extracellular portion of an α chain or β chain and a DNA coding for the constant region of human immunoglobulin heavy chain are linked with in frame. The obtained DNA codes for a polypeptide starting from the methionine of translation initiation and having the signal sequence of the α chain or β chain of an integrin, its extracellular region and the constant region of human immunoglobulin heavy chain linked in this order.

The DNA coding for a chimeric protein consisting of the α chain of an integrin and the heavy chain or light chain of an immunoglobulin, or the DNA coding for a chimeric protein consisting of the β chain of an integrin and the heavy chain or light chain of an immunoglobulin respectively obtained in the above is functionally linked in a proper expression control sequence, to obtain a recombinant vector. The general methods concerning gene recombination such as the method for preparing the recombinant vector, the method for transfecting it into a cell are described in a published book ("Molecular Cloning", Sambrook et al., (1989) Cold Spring Harbor Lab. Press, New York), but the methods are not limited to those stated there. In the present invention, it is desirable to use an expression control sequence suitable for protein expression in animal cells. For example, for manifestation of insect cells, polyhedrin promotor, p10 promotor, etc. are generally used as expression control sequences, and for expression of other animals' cells, SRα promotor, cytomegalovirus derived promotor, simian virus 40 derived promotor, polyhedrin promotor, p10 promotor, etc. are used. However, the expression control sequences are not limited to them. In the present invention, it is preferable to use SRα promotor.

If the obtained recombinant vector is transfected into a cell, a cell capable of producing an integrin-immunoglobulin chimeric protein heterodimer complex can be obtained. In this case it is preferable to use an animal derived cell as a host. For example, COS cell (simian renal cell), CHO cell (Chinese Hamster ovarian cell), Sf9 (insect cell), etc. are generally used as hosts. Furthermore, myeloma cells such as P3U1 and Y3 can also be used. Other established cell lines and cloned cells can also be used, but the cells used as hosts are not limited to them. In the present invention, it is preferable to use a CHO cell.

It is known that the methods for transfecting a recombinant vector into a cell include the lipofectin method, calcium phosphate method, electroporation method, etc., and any of the methods can be used. The method is not limited to them. It is preferable that when a cell is transfected by using a recombinant vector, a recombinant vector for expression of a chimeric protein consisting of the α chain of an integrin and the heavy chain or light chain of an immunoglobulin and a recombinant vector for expression of a chimeric protein consisting of the β chain of the integrin and the heavy chain or light chain of the immunoglobulin are transfected into the cell one after another using different drug resistance markers. The recombinant vectors can be transfected in any order or simultaneously. It is desirable that the two recombinant vectors to be transfected are vectors for expression of a combination consisting of α chain·immunoglobulin heavy chain (which means a chimeric protein consisting of an α chain and the heavy chain of an immunoglobulin; hereinafter this applies) and β chain·immunoglobulin heavy chain, or α chain·immunoglobulin heavy chain and β chain·immunoglobulin light chain, or α chain·imminoglobulin light chain and β chain·immunoglobulin heavy chain. Any of these combinations can be adopted, but a combination of recombinant vectors for expression of α chain·immunoglobulin heavy chain and β chain·immunoglobulin heavy chain is desirable.

In any transfection method and any combination of vectors, it is important to select a cell which is transfected by the two recombinant vectors and produces a chimera protein consisting of an α chain and the heavy chain or light chain of an immunoglobulin and a chimera protein consisting of a β chain and the heavy chain or light chain of an immunoglobulin simultaneously almost by the same amounts. This can be achieved by measuring the amounts of the chimeric protein consisting of an α chain and the heavy chain or light chain of an immunoglobulin and a chimeric protein consisting of a β chain and the heavy chain or light chain of an immunoglobulin produced in the cultured supernatant solution of the cell transfected by the recombinant vectors. For measurement, for example, the transfected cell can be cultured in a medium containing ³⁵S according to any publicly known method, for labeling the proteins, and the amounts of the chimeric protein consisting of an α chain and the heavy chain or light chain of an immunoglobulin and a chimeric protein consisting of a β chain and the heavy chain or light chain of an immunoglobulin existing in the cultured supernatant solution can be estimated by immunoprecipitation using an anti-α chain antibody or an anti-β chain antibody respectively. As another method, the amounts the chimeric protein consisting of an α chain and the heavy chain or light chain of an immunoglobulin and a chimeric protein consisting of a β chain and the heavy chain or light chain of an immunoglobulin existing in the cultured supernatant solution can be estimated according to the ELISA method using an anti-human immunoglobulin antibody and an anti-α chain antibody or an anti-β chain antibody. Anyway, it is preferable to select a clone which produces almost the same large amounts of the chimeric proteins of the α and β chains in the culture supernatant , for preparing an integrin-immunoglobulin chimeric protein heterodimer complex. The methods for labeling proteins, the methods of immunoprecipitation and the general methods of ELISA are described in a published book ("Antibody" Harlow, E., and Lane, D. (1988), Cold Spring Harbor Lab. Press, New York), but the methods are not limited to them. Any other method can also be used for detecting chimeric proteins.

The obtained transfected cell can be cultured according to a general cell culture method, to produce an integrin-immunoglobulin chimeric protein heterodimer complex. It is preferable that the medium contains about 5% of serum of a low immunoglobulin concentration, but any generally known serum-containing medium or a serum-less medium can also be used. After completion of cell culture, the cells and solid matter are removed by such operation as centrifugation, and the culture supernatant containing an integrin-immunoglobulin chimeric protein heterodimer complex is collected.

It can be estimated that the cultured supernatant solution contains not only the integrin-immunoglobulin chimeric proteins in which the chimeric protein consisting of an α chain and the heavy chain or light chain of an immunoglobulin and a chimeric protein consisting of a β chain and the heavy chain or light chain of an immunoglobulin form a heterodimer complex, but also the chimeric protein consisting of an α chain and the heavy chain or light chain of an immunoglobulin and a chimeric protein consisting of a β chain and the heavy chain or light chain of an immunoglobulin which do not form the heterodimer complex. However, since the molecules other than the heterodimer complex cannot be bound to a ligand, the supernatant solution can be used as a reagent for testing the binding to a ligand or cell, or searching for a substance inhibiting the binding between an integrin and a ligand, or for searching for a substance capable of being bound to an integrin, or for measuring the ligand amount of an integrin. These methods of utilization are basically the same as those for using a purified integrin-immunoglobulin chimeric protein heterodimer complex described later.

An integrin-immunoglobulin chimeric protein heterodimer complex can be purified by an established method using a protein A column chromatography by use of the nature of the immunoglobulin portion. Furthermore, affinity chromatography using an antibody against the α or β chain can a so be used. Moreover, the purification can also be achieved by affinity chromatography with a ligand bound to a carrier. General chromatographic methods can also be used in combination for the purification. If publicly known cases in which integrin molecules are purified by these methods (Pytela, R. et al., Methods Enzymol., 144, 475-489 (1987), Santoro, S. A. et al., Biochem. Biophys. Res. Comm., 153, 217-223 (1988), Charo, I.F. et al., J. Cell Biol., 111, 2795-2800 (1990), Makarem, R. et al., J. Biol. Chem., 269, 4005-4011 (1994), Pfaff, M. et al., Eur. J. Immunol., 225, 975-984 (1994), Gulino, D. et al., Eur. J. Biochem., 227, 108-115 (1995), etc.) are applied, the purification of an integrin-immunoglobulin chimeric protein heterodimer complex can be achieved.

A purified integrin-immunoglobulin chimeric protein heterodimer complex can be identified as a protein showing at least one band under non-reducing condition and at least two bands under reducting condition by SDS-PAGE. It can also be confirmed from it, that the heterodimer is linked by the disulfide bond between immunoglobulin heavy chains. It sometimes occurs that a plurality of bands are detected under reduction, but this is considered to be probably because intramolecular clearage of the α chain has occurred. Especially, with α4, this phenomenon is known (Hemler, M. E. et al., J. Biol., Chem., 262, 11478-11485 (1987)). Furthermore, it can be confirmed by the Western blotting method that the respective bands indicate chimeric proteins. As another method, it can be confirmed by said ELISA method combining an anti-α chain anti body, anti-β chain antibody and anti-human immunoglobulin antibody, that the obtained molecule is an integrin-immunoglobulin chimeric protein heterodimer complex. That is, the molecule can be identified as a protein molecule with epitopes for all the antibodies. As a further other method, an integrin-immunoglobulin chimeric protein heterodimer complex can also be identified by immunoprecipitation. In this case, if the purified protein is labeled by ³⁵S, or ¹²⁵I or biotin, etc. according to any known method, and immunoprecipitated using an anti-α chain antibody, anti-β chain antibody and anti-human immunoglobulin antibody, the same electrophoretic pattern can be obtained in every case. So, it can be confirmed that the integrin-immunoglobulin chimeric protein heterodimer complex has the intended structure. Furthermore, even if a condition to dissociate the integrin complex on a cell membrane such as the coexistence of EDTA or boiling in the presence of SDS is applied, the immunoprecipitation pattern is not changed. So, it can be confirmed that the obtained integrin-immunoglobulin chimeric protein heterodimer complex is a structurally stabilized complex. The methods for confirming an integrin-immunoglobulin chimeric protein heterodimer complex are not limited to those stated above.

The binding between a prepared integrin-immunoglobulin chimeric protein heterodimer complex and a ligand can be tested as described below. After a ligand and an integrin-immunoglobulin chimeric protein heterodimer complex are brought into contact with each other, to make a mixture, the amount of the integrin-immunoglobulin chimeric protein heterodimeter complex bound to the ligand or the amount of the ligand bound to the integrin-immunoglobulin chimeric protein heterodimeter complex is measured. The amount of an integrin-immunoglobulin chimeric protein heterodimer complex can be measured by labeling the complex itself by a fluorescent dye or enzyme or radio isotope, etc. The amount of a ligand can also be measured by any similar method. A detection method such as SPA (Amasham) can also be used for the measurement. Furthermore, any reagent which can recognize a complex or ligand labeled by a fluorescent dye, enzyme or radioisotope, etc. can also be used for the measurement. The reagent for recognizing an integrin-immunoglobulin chimeric protein heterodimer complex can, for example, be an anti-human immunoglobulin antibody. In this test, it is preferable to bind the molecule to be detected, to any carrier such as a bead or plate. As a ligand, its entire molecule can be used, but a portion retaining the binding activity to an integrin can also be taken out for use. For example, for integrin α4β1 or integrin α2β1, its ligand, fibronectin or collagen or its peptide fragment bound to a carrier can also be used.

Methods similar to the above can be used to test the binding between an integrin-immunoglobulin chimeric protein heterodimer complex and cells. The amount of the cells bound to a complex can be measured by labeling the cells by a fluorescent dye or radioisotope or using a reagent reacting with the cells, for example, an antibody reacting with a surface antigen. If something like a tissue section is used instead of cells, the amount of the bound integrin-immunoglobulin chimeric protein heterodimer complex is measured by any of the above mentioned methods.

The methods for examining the binding between an integrin-immunoglobulin chimeric protein heterodimer complex and a ligand or cell described above can be used for obtaining a substance inhibiting the binding between an integrin and a ligand, for example, for obtaining an antibody, polypeptide, peptide or low molecular weight compound. It is preferable to premix a sample and an integrin-immunoglobulin chimeric protein heterodimer complex, and then to measure the amount of the integrin-immunoglobulin chimeric protein heterodimer complex bound to a ligand in any of the above mentioned measuring systems. If the amount of the bound integrin-immunoglobulin chimeric protein heterodimer complex is lowered by adding a certain sample, it can be judged that the sample has inhibitory activity. However, in this sytem, a substance with metal ion cheating action or a substance with surfactant action, etc. may give a false positive result. The sources of samples used include the following integrin bound substances, peptide fragments of ligands, their derivatives, marketed compounds, etc., but are not limited to them.

A case where a purified integrin was coated on a plate to search for a peptide to be bound was reported (Healy, J. M. et al., Biochemistry 34, 3948-3955 (1995)). Even if the integrin-immunoglobulin chimeric protein heterodimer complex obtained in the present invention is used, a substance to be bound to an integrin can be similarly searched for. Especially when the chemiric protein heterodimer complex of the present invention is used, the operation to remove the non-specifically bound substances can be effected under more severe conditions. So, the operation can be simplified advantageously. Furthermore, since the complex is not dissociated during operation, a bound substance can be selected more specifically advantageously. Known sources suitable for selecting bound substances include a phage peptide library (e.g., Scott, J. K. and Smith, G. P., Science, 249, 386-390 (1990)) and a DNA oligomer library (e.g., O'Connel, D. et al., Proc. Natl. Acad. Sci. USA, 93, 5883-5887 (1996), but in the present invention, it is preferable to use the former.

Furthermore, the method of testing the binding between an integrin-immunoglobulin chimeric protein heterodimer complex and a ligand or cell can also be used as a method for measuring the amount of an integrin ligand in a body fluid or tissue.

Moreover, the integrin-immunoglobulin chimeric protein heterodimer complexes of the present invention can also be used as drugs. The present invention has clarified that integrins and other isolated extracellular matrix receptors can be used as platelet substitutes.

An extracellular matrix receptor preferably used as a platelet substitute is an integrin. The α chain of the integrin can be α1, α2, α3, α4, α5, α6, α7, α8, α9, αv, αL, αM, αX, αIIb or αE, and among them, α2 is preferable. The β chain can be β1, β2, β3, β4, β5, β6, β7or β8, and among them, β1 is preferable. Integrin α2β1 is more preferable. The receptor source for isolation can be a tissue or cell expressing an extracellular matrix receptor, or a dissolved membrane fraction of a receptor expressing cell prepared by gene manipulation, etc. It is more preferable to design for obtaining a soluble protein by modifying a receptor gene by gene recombination, and to use the cultured supernatant solution of the cells capable of producing it, as a source. Furthermore in the design of the soluble protein, it is preferable that the functional structure of the extracellular matrix receptor is retained. For example, it is desirable to use an integrin-immunoglobulin chimeric protein heterodimer complex obtained by modifying the heterodimer structure of an integrin to allow its α and β chains to be covalently associated with each other. As the integrin-immunoglobulin chimeric protein heterodimer complex, it is preferable that the α chain of the integrin is α1, α2, α3, α4, α5, α6, α7, α8, α9, αv, αL, αM, αX, αIIb or αE, and among them, α2 is more preferable. Furthermore, it is preferable that the β chain is β1, β2, β3, β4, β5, β6, β7 or β8, and among them, β1 is more preferable. It is further more preferable that the α chain is α2 and that the β chain is β1. The platelet substitute of the present invention is described below mainly in reference to a typical extracellular matrix receptor, integrin α2β1-immunoglolublin chimeric protein heterodimer complex, but the present invention is not limited thereto or thereby.

To confirm the applicability of a purified integrin-immunoglobulin chimeric protein heterodimer complex as a drug, the purified protein itself is used for examining its pharmacological activity. For obtaining higher capability of being bound to an extracellular matrix, it is more preferable to use an integrin-immunoglobulin chimeric protein heterodimer complex bound to a carrier such as a lipid or protein polymer, etc., but the present invent ion is not limited to this method.

For use as a platelet substitute, it is preferable to bind an integrin α2β1-immunoglolublin chimeric protein heterodimer complex to a liposome covalently according to the method stated in a report (Martin, F. J. et al., Biochemistry, 20, 4229 (1981)). The carrier can also be any other drug carrier than a liposome as far as its use for drugs is permitted. If a liposome is used as the carrier, the liposome is prepared according to the composition and method stated in a published book "Preparation and Experiments of Liposomes (in Japanese)", Oku, N. (1994), Hirokawa Shoten), but a preferable method is such that the epitope bound to the extracellular matrix of an integrin α2β1-immunoglolublin chimeric protein heterodimer complex is exposed outside the liposome membrane.

For confirming that an integrin α2β1-immunoglolublin chimeric protein heterodimer complex is bound on the prepared liposome carrier, a flow cytometer is used. The reagents which can be used for recognizing the integrin α2β1-immunoglolublin chimeric protein heterodimer complex include an anti-integrin α2 antibody, anti-integrin β1 antibody, anti-human immunoglobulin antibody, etc. If the antibody used is fluorescently labeled, it can be used for determination directly, but if it is not fluorescently labeled, a secondary antibody which recognizes the immunoglobulin class of the animal species used for preparing the antibody is used as a fluorescent label. As a further other confirmation method, the integrin α2β1-immunoglolublin chimeric protein heterodimer composite itself can be labeled by an enzyme or radioisotope, etc., for confirmation in proper combination with a color dye or radioactivity measuring instrument, etc.

To examine the extracellular matrix binding capability using an integrin α2β1-immunoglolublin chimeric protein heterodimer complex liposome, it is preferable to suspend the integrin α2β1-immunoglolublin chimeric protein heterodimer complex liposome into a buffer with a physiological cation concentration or plasma. The buffer with a physiological cation concentration refers to a buffer containing at least cations such as Mg ions or Ca ions and adjusted to about neutrality. The plasma is prepared by processing the blood collected in the presence of an anticoagulant, according to a general plasma preparation method. As the anticoagulant, for example, heparin or EDTA solution can be added by sufficient units. A marketed normal plasma, coagulation factor deficient plasma or serum, etc. can also be used. However, if the anticoagulant used lowers the cation concentration, cations are added to achieve a physiological concentration later. Then, the integrin α2β1-immunoglolublin chimeric protein heterodimer complex liposome is mixed with an extracellular matrix or its fragment coated on a carrier for a certain time, to judge whether binding takes place. It is preferable that the coating of the extracellular matrix or its fragment as a solid phase is achieved by using a plastic plate, etc., but marketed beads for coating an extracellular matrix as a solid phase, etc. can also be used. When a collagen is used as the extracellular matrix, any animal species and type can be used. The binding reaction between an integrin α2β1-immunoglolublin chimeric protein heterodimer complex liposome and an extracellular matrix is effected according to a general method adopted for observing the adherence reaction of platelets. In many cases, they are allowed to stand mainly in a static system for a certain time, to induce binding to the matrix, but it is preferable to apply a shaking or shear stress, etc.

The integrin α2β1-immunoglolublin chimeric protein heterodimer complex liposome is bound to an extracellular matrix under the conditions as described above, and the amount of binding is measured by applying the above mentioned ELISA method using an anti-human immunoglobulin antibody. For more accurate determination, it is desirable to immobilize the liposome bound to the matrix by 1% glutaraldehyde, etc. As another method than the ELISA method, for example, if a radio-labeled lipid is incorporated into the liposome beforehand, the amount of the liposome bound to the extracellular matrix can be obtained as radioactivity. Furthermore, to qualitatively judge the binding and covering degree to the extracellular matrix, a labeled antibody for recognizing the integrin α2β1-immunoglolublin chimeric protein heterodimer complex on the bound liposome can be combined with a color dye, etc., to dye the portions where the liposome is bound. It is more preferable that the generally used tissue antibody dyeing method is used to use a peroxidase labeled antibody against the integrin α2β1-immunoglolublin chimeric protein heterodimer complex and diaminobenzidine in combination, but the measuring method is not limited to it. As a further other method, the area covering the extracellular matrix can be obtained as a covering rate using an image processing analyzer.

Methods for examining the hemostasis of platelets include testing the adhering capability of platelets to the extracellular matrix and the agglutination capability induced by a collagen ("Handbook on the Examination of Blood Coagulation (in Japanese)", p. 65-78, Fukutake, M. and Fujimaki, M. (1987), Uchudo Yagi Shoten, Santro, S.A., Cell, 46, 913-920 (1986), Lethagen, S. and Rugarrn, P., Thrombo Haemost., 67, 185-186 (1982)). Especially the adhering capability of platelets to the extracellular matrix is an indicator of primary hemostasis. The adhering capability is evaluated by using blood as it is, or platelet rich plasma or platelets washed by a buffer with physiological ions. Therefore, whether or not the integrin α2β1-immunoglolublin chimeric protein heterodimer complex liposome obtained in the present invention can be a functional substitute of platelets can be judged in reference its binding capability and the level of the binding capability to the extracellular matrix in the existence of plasma components or at a physiological ion concentration.

If the binding capability of the integrin α2β1-immunoglolublin chimeric protein heterodimer complex liposome obtained in the present invention to the extracellular matrix in the presence of the plasma components is strong, it suggests that the liposome can be a platelet substitute. Therefore, it can be used as a therapeutic or preventive agent against the congenital and acquired bleeding tendency due to platelet abnormality, and also widely as a platelet transfusion substitute.

Similarly the integrin α2β1-immunoglolublin chimeric protein heterodimer complex liposome obtained in the present invention can be a therapeutic or preventive agent for conditions of diseases where vascular endothelial cell disorder is a problem. For example, it was reported that in the prognosis of PTCA (percutaneous coronary restenosis), the excessive accumulation of platelets on the extracellular matrix exposed by balloon catherter treatment triggers restenosis (Liu, M.W. et al., Circulation, 79, 1374-1378 (1989)). In Example 22, the effect of the integrin α2β1-immunoglolublin chimeric protein heterodimer complex liposome to cover the extracellular matrix was confirmed, and this effect can reduce the excessive accumulation of platelets to allow use also as a restenosis preventive. Furthermore, if the integrin α2β1-immunoglolublin chimeric protein heterodimer complex liposome is labeled by a medically allowable method, it can be used for monitoring the region of the extracellular matrix exposed by vascular endothelial cell injury, and furthermore, if a drug is enclosed in the liposome, it can also be applied to the targeting therapy for a local injured region.

When any integrin α2β1-immunoglolublin chimeric protein heterodimer complex liposome stated in the present invention is used as a platelet substitute, the administration paths include infusion, intravenous administration, etc., and it is usually used by being suspended in any physiologically suitable solution such as a salt solution or plasma, etc. It can be used alone or also in combination with another chimeric protein heterodimer complex with an extracellular matrix receptor or its immunoglobulin. It can also be used together with another drug containing total platelets. The dose is properly selected to suit the symptom, age, body weight, etc. and can be 0.1 mg to 10 g per day as the amount of the protein for an adult, being able to be administered at a time or in several times. It can also be mixed with a pharmaceutically allowed carrier or excipient, etc., to be applied locally to the injured region as an externally applied drug such as an ointment, liniment or plaster. In this case, the externally applied drug is prepared to be 1 ng/cm² to 1 mg/cm² as the amount of the protein per one time of coating.

### Examples

To describe the present invention in more detail, examples are given below. The general methods of recombinant DNA experiments conformed to those stated in a published book ("Antibody", Harlow, E. and Lane, D. (1988), Cold Spring Harbor Lab. Press, New York).

### Example 1

### Construction of human IgG ₁ heavy chain expression vector

As human IgG ₁ genome gene, a clone identical with reported base sequence information (Ellison, J. W. et al., Nucleic Acids Res., 10, 4071-4079 (1982)) was acquired from a human genomic library (CLONTECH) using a hybridization cDNA probe based on the sequence information. This was used as the template DNA for PCR. As primers for amplifying the DNA fragment containing the hinge region (H) and the constant region portions (CH2 and CH3) of human IgG₁ gene, a DNA oligomer shown in sequence No. 4 of the sequence table (hereinafter a sequence No. of the sequence table is simply called a sequence No.) with BamH I restriction site and a DNA oligomer shown in sequence No. 5 with Xba I restriction site were synthesized.

5'-GCGGATCCCGAGCTGCTGGAAGCAGGCTCAG-3'    (Sequence No. 4)
5'- CCTCTAGACGGCCGTCGCACTCATTTA-3'    (Sequence No. 5)

The template DNA, primers, dNTPs (an equimolar mixture of dATP, dCTP, dGTP and dTTT) and Taq polymerase (Takara) were mixed in a PCR buffer (100 mM Tris-HCl, 500 mM KCl, 15 mM MgCl₂, 0.01% gelatin, pH 8.3), and in a thermal cycler (Perkin Elmer Cetus), the mixture was treated at 94 °C for 1 minute for DNA denaturation, at 58°C for 2 minutes for annealing the primers and at 72°C for 3 minutes for elongating the primers. This treatment was performed 30 cycles. The amplified DNA was digested by restriction enzymes BamH I and Xba I, and the DNA fragment was purified by 1% agarose gel according to a general method ("Antibody", Harlow, E. and Lane, D. (1988), Cold Spring Harbor Lab. Press, New York). It was linked, using a T4DNA ligase, with a large DNA fragment of pBluescriptSK(+) (STRATAGENE) purified and digested by restriction enzymes BamH I and Xba I. The plasmid DNA was used to transform Escherichia coli (JM109), and the transformant was selected, to obtain a plasmid DNA (IgG₁ Bluescript). Then, expression vector pcDL-SR α296 was digested by restriction enzyme BamH I, and blunted at the termini by T4DNA polymerase treatment, and a Not I linker was linked. The large DNA fragment obtained by digesting it by restriction enzymes Not I and Xho I and the small DNA fragment obtained by digesting IgG₁Bluescript by restriction enzymes Not I and Xho I were purified according to a general method, and linked by T4DNA ligase. It was transformed into Escherichia coli (HB101) , and the transformant was selected, to obtain a plasmid DNA. Hereinafter this plasmid (IgG ₁SRα) is called human IgG₁ expression vector. In the following examples, since the basic protocol of gene manipulation is the same as above, the description will be simplified.

### Example 2

### Construction of integrin α4·IgG heavy chain chimeric protein expression vector

The DNA fragment coding for the extracellular portion of integrin α4 was obtained by cloning based on reported cDNA sequence information (Takada, Y. et al., EMBO J., 8, 1361-1368 (1989)). The restriction site EcoR I of 1801-base-position of sequence No. 1, the restriction site Stu I of 112-base-position and the restriction site BamH I of 2949-base-position were used for linking the region from the N terminus translation initiation site to Stu I cut site as α4-1, the region from Stu I cut site to EcoR I cut site as α4-2, and the region from EcoR I detached site to BamH I detached site as α4-3. The detailed methods are described below.

The portion coding for α4-1 was designed to be cloned by linking the DNA oligomers of sequence Nos. 6 to 9, and the DNA oligomers shown in sequence Nos. 6 to 9 were synthesized. For the sequence Nos. 6 and 7, restriction site Xba I was added on the side to code for the N terminus, for linking to a vector. Furthermore, compared with the known sequence information, the bases at the 60-, 63- and 64-positions were substituted from C to T, C to A and C to G respectively, and the bases at the 112- and 114-positions were substituted frseom C to A and C to G respectively. Because of substitution at the 112- and 114-position, restriction site Stu I was inserted on the side to code for the N terminus of sequence Nos. 8 and 9. The 5' termini of the synthesized oligomers were phosphated and annealed, and were linked using T4DNA ligase. After completion of linking, restriction enzymes Xba I and Stu I were used for cutting, and electrophoresis was effected by 5% agarose (NuSieve GTGagarose, FMC) gel. The intended DNA fragment (α4-1) of about 120 bp was cut out and purified.

5' - CTAGACCACCATGTTCCCCACCGAGAGCGCATGGCTTGGGAAGCGAGGCGCGAACCCGGCCCCGGA
GCTGCA-3'
(Sequence No. 6)
5' - GCTTCGGGGCCCGGGTTCGCGCCTCGCTTCCCAAGCCATGCGCTCTCGGTGGGGAACATGGTGGT-3'
(Sequence No. 7)
5' - CTCCGGGAGACGGTGATGCTGTTGCTGTGCCTGGGGGTCCCGACCGCCAGG-3'

(Sequence No. 8)
5'-CCTGCCGGTCGGACCCCCAGGCACAGCAACAGCATCACCGTCTCCCGGAGTCGA-3'
(Sequence No. 9)

Then, the RNA of human osteosarcoma cell line MG63 (ATCC CRL 1427) as an integrin α4 expressing cell was separated, and PolyA(+)RNA was purified using oligo dT cellulose column (NEB). Based on it, a single stranded cDNA was synthesized using a reverse transcriptase (GIBCO), and used as the template for PCR. As primers for amplifying α4-2 and α4-3 DNAs, four DNA oligomers of sequence Nos. 10 to 13 with Pst I and Stu I restriction sites inserted (sequence No. 10) or BamH I restriction site inserted (sequence No. 13) were synthesized.

5'- CACTGCAGGCAGGCCTTACAACGTGGACACTGAGAGC-3' (Sequence No. 10)
5'- GCAGAAACCTGTAAATCAGCAG-3' (Sequence No. 11)
5'- GCATTTATGCGGAAAGATGTGC-3' (Sequence No. 12)
5'- CGGGATCCGTGAAATAACGTTTGGGTCTT-3' (Sequence No. 13)

The template cDNA, primers, dNTPs and Taq polymerase were mixed in a PCR buffer, and the mixture was treated by a thermal cycler at 94°C for 1 minute for DNA denaturation, at 58°C for 2 minutes for annealing the primers and at 72°C for 3 minutes for elongating the primers. This treatment was performed 30 cycles. The amplified DNA fragments of α4-2 and α4-3 were digested by Pst I and EcoR I respectively, or EcoR I and BamH I and sub-cloned into pBluescriptKS(+) (STRATAGENE), to prepare plasmid DNAs (hereinafter called α4-2 Bluescript and α4-3 Bluescript). Then, upstream of the α4-2 Bluescript, α4-1 was linked using Xba I and Stu I restriction sites, to prepare a plasmid DNA (hereinafter called α4-1-2 Bluescript).

The α4-1-2 Bluescript was digested by restriction enzyme Not I, and blunted at the termini by T4DNA polymerase treatment, being digested by restriction enzyme EcoR I, to prepare a small DNA fragment. The α4-3 Bluescript was digested by restriction enzymes EcoR I and BamH I, to prepare a small DNA fragment. The two small DNA fragments were simultaneously linked to a large DNA fragment obtained by digesting IgG₁SRα by restriction enzymes EcoR V and BamH I, to obtain a plasmid DNA. The obtained base sequence coding for integrin α4·IgG heavy chain chimeric protein is shown as sequence No. 1. The plasmid (integrin α4·IgGSRα) is hereinafter called integrin α4·IgG heavy chain chimeric protein expression vector.

### Example 3

### Construction of β1·IgG heavy chain chimeric protein expression vector

The RNA of human fibroblast cell line MRC5 (ATCC CCL 171) as an integrin β1 expressing cell was separated, and oligo dT cellulose column was used to purify PolyA (+)RNA. Based on it, a single stranded cDNA was synthesized using a reverse transcriptase, and used as the template for PCR. As primers, two DNA oligomers of sequence Nos. 14 and 15 with BamH I site (sequence No. 15) inserted on the side coding for C terminus were synthesized according to the sequence information (Cott Argraves, W. et al., J. Cell Biol., 105, 1183-1190 (1987)).

5'-GCGGAAAAGATGAATTTACAAC-3'          (Sequence No. 14)
5'-GTGGGATCCTCTGGACCAGTGGGACAC-3'     (Sequence No. 15)

The template cDNA, primers, dNTPs and Taq polymerase were mixed in a PCR buffer, and treated by a thermal cycler at 94°C for 1 minute for DNA denaturation, at 57°C for 2 minutes for annealing the primers and at 72°C for 3 minutes for elongating the primers. This treatment was performed 30 cycles. The amplified DNA was blunted at the termini by T4DNA polymerase treatment, and digested by restriction enzyme BamH I. Then, the DNA fragment was purified. Subsequently, the DNA fragment obtained in PCR before was sub-cloned at the Sma I and BamH I sites of pBluescriptKS(+). A small DNA fragment purified by digesting it by restriction enzymes EcoR I and BamH I was inserted into a large DNA fragment of IgG₁SRα treated by restriction enzymes EcoR I and BamH I, to obtain a plasmid DNA. The obtained base sequence coding for β1·IgG heavy chain chimeric protein is shown in sequence No. 2. The plasmid (integrin β1·IgGSRα) is hereinafter called integrin β1·IgG heavy chain chimeric protein expression vector.

### Example 4

### Transfection of α4·IgG heavy chain chimeric protein expression vector and β1·IgG heavy chain chimeric protein expression vector into animal cells, and their expression

Integrin β1·IgGSRα as β1·IgG heavy chain chimeric protein expression vector and pSV2dhfr (BRL) were mixed at a ratio of 10 : 1, and the mixture and lipofectin reagent (GIBCO BRL) were gently mixed and allowed to stand at room temperature for 15 minutes. The mixture was added dropwise to dihydrofolic acid reductase deficient CHO cells (ATCC CRL 9096). After 18 hours of dropwise addition, the mixture was cultured in a medium (10%FBS (GIBCO), nucleic acid-containing αMEM medium (GIBCO BRL)) for about 2 days, and the cells were dispersed by trypsin-EDTA treatment. The cells were suspended in a first selective medium (10% FBS-containing nucleic acid-free αMEM medium (GIBCO BRL)), and the suspension was disseminated into a 96-well plate (CORNING), for selective culture for about 10 days. Then, the amount of integrin β1·IgG heavy chain chimeric protein produced in the culture supernatant was determined according to the ELISA method (described later), and the clone showing the highest production was stabilized by cloning according to the limiting dilution method.

Then, into the stabilized integrin β1·IgG heavy chain chimeric protein producing CHO cells, the integrin α4·IgG heavy chain chimeric protein expression vector was transfected according to the lipofectin method as described before. That is, integrin α4·IgGSRα and pSV2neo (BRL) were mixed at 10 : 1, and the mixture was mixed with lipofectin reagent. The mixture was added dropwise into the cells. After 18 hours of dropwise addition, the mixture was cultured in the said first selective medium for about 2 hours, and the cells were dispersed by trypsin-EDTA treatment. The cells were suspended in a second selective medium (nucleic acid-free αMEM medium (GIBCO BRL) containing 10% FBS (GIBCO) and 1 mg/ml neomycin (GIBCO)), and on a 96-well plate (CORNING), resistant cells were selectively cultured for about 10 days. The amount of integrin α4·IgG heavy chain chimeric protein and the amount of integrin β1·IgG heavy chain chimeric protein produced in the culture supernatant were determined according to the ELISA method (described later), and a clone which produced both the chimeric proteins by almost the sane amounts was picked up. The clone was cloned twice according to the limiting dilution method, to be stabilized as a clone capable of producing α4·IgG heavy chain-β1·IgG heavy chain chimeric protein heterodimer complex.

### Example 5

### Determination of produced integrin α4·IgG heavy chain chimeric protein and integrin β1·IgG heavy chain chimeric protein by the ELISA method

Fifty microliter per well of anti-human integrin α4 antibody (Becton & Dickinson, Clone L25.3) or anti-human integrin β1 antibody (Coulter, Clone 4B4) (12 µg/ml each) was put into a 96-well immunoplate (NUNC) , and allowed to stand at 4°C for 16 hours. Then, each well was washed by Dulbecco's phosphate buffered saline (Nissui Seiyaku, not containing Ca or Mg ions, hereinafter called PBS(-)) twice, and non-specific reaction was blocked by PBS(-) containing 25% Block Ace (Snow Brand Milk Products Co., Ltd.). After blocking, the culture supernatant of CHO cells grown in selective medium was properly diluted, and reacted with the coated antibody at room temperature for 1 hour. After the reaction, the surface of the plate was washed with 0.02% Tween-containing PBS(-) (hereinafter called T-PBS) twice. It was then caused to react with biotinated anti-human IgG antibody (Vector) for 1 hour, and the reaction mixture was washed with T-PBS twice, and in succession caused to react with avidin-horseradish peroxidase (Sigma) for 1 hour. The reaction mixture was washed with PBS(-) twice. The PBS(-) was perfectly aspirated, and or thophenylenediamine was used as a substrate for color development. The absorbance at 490 nm were measured using a microplate reader (Bio-rad NOVAPATH), and the clone showing a high absorbance value was selected.

### Example 6

### Purification of α4·IgG heavy chain-β1·IgG heavy chain chimeric protein heterodimer complex

### (1) Culture of CHO cells and preparation of cultured supernatant solution

The CHO cells highly capable of producing the α4·IgG heavy chain-β1·IgG heavy chain chimeric protein heterodimer complex were cultued in nucleic acid-free αMEM medium containing 5% FBS (Ultra-low IgG grade, GIBCO) (hereinafter called αMEM(-) medium, GIBCO BRL) for one day, to reach semiconfluent, and they were cultured in αMEM(-) medium containing 1% FBS (Ultra-low IgG grade) for 3 days, and the culture supernatant was collected. It was concentrated to 1/10 volume by ultrafiltration using Prep-scale (Millipore), and 1M Hepes solution (pH 8.0) was added to achieve a final concentration of 5 mM, for preparing a starting solution for further purification.

### (2) Protein A column chromatography

The starting solution for further purification was passed through Prosep Guard column (bioPROCESSING) , and applied to Prosep A column (bioPROCESSING) . After completion of application, it was washed with 10 times the column volume of PBS(-), and the proteins were eluted at a pH 6 ⁻ 3 gradient of 0.1M citrate buffer solutions. The peak fraction eluted at pH 3 was collected, and 1M Tris-HCl solution (pH 8.5) was added by 0.1 volume for neutralization. The solution was dialyzed against PBS(-).

### (3) Affinity column chromatography

FMP activated Cellulofine (Seikagaku Kogyo) was equilibrated by a coupling buffer (50 mM Na₂CO₃-NaHCO₃ pH 8.5), and a peptide showing sequence No. 3 (hereinafter called CS-1 peptide) synthesized by a peptide synthesizer was added. The mixture was inverted and mixed at 4 °C for 16 hours. Cys Leu His Gly Pro Glu Ile Leu Asp Val Pro Ser Thr (Sequence No. 3) After completion of mixing, the mixture was washed with the coupling buffer, and a blocking buffer (0.1 mM monoethanolamine, 50 mM Tris-HCl, pH 8.0) was added. The mixture was inverted and mixed further at room temperature for 6 hours. Then, the mixture was suffificiently washed with TBS solution (150 mM NaCl, 20 mM Tris-HCl, 1 mM MnCl₂, pH 7.5), to prepare CS-1 peptide bound Cellulofine column. To the column the starting solution for further purification was applied and allowed to stand at room temperature for 3 hours, and washed with 10 times the column volume of a washing buffer (1M NaCl, 0.1% Triton, 20 mM Tris-HCl, 1 mM MnCl ₂, pH 7.5) and the same volume of the TBS solution. After completion of washing, an elution buffer (10 mM EDTA, 150 mM NaCl, 20 mM Tris-HCl, pH 7.5) was used, to elute the proteins bound to the CS-1 column. The eluate was collected and dialyzed against PBS(-).

### (4) SDS-PAGE

The eluted fractions of (3) were subjected to SDS-PAGE under non-reducing or reducing condition using 6.0 or 7.0% acrylamide gel, and the gel was stained with Coomassie-blue. As a result, under non-reducing condition, two bands considered to be attributable to the α4·IgG heavy chain-β1·IgG heavy chain chimeric protein heterodimer complex and its polymer were observed. Under reducing condition, two bands (170 kDa and 135 kDa) considered to be attributable to the integrin α4·IgG heavy chain chimeric protein and the integrin β1·IgG heavy chain chimeric protein and two bands (80 kDa and 90 kDa) considered to be attributable to the intramolecular cleavage of the integrin α4·IgG heavy chain chimeric protein (Hemler, M. E. et al., J. Biol. Chem., 262, 11478-11485 (1987)) were observed. These results suggest that the eluted protein of (3) has a molecular structure considered to be α4·IgG heavy chain-β1·IgG heavy chain chimeric protein heterodimer complex, and that the molecules constituting the heterodimer are linked by a disulfide bond between the IgG heavy chains.

### Example 7

### Identification of α4·IgG heavy chain-β1·IgG heavy chain chimeric protein heterodimer complex, and examination of its structural stability

### (1) Immunoprecipitation using anti-integrin antibodies and influence of a cationic chelating agent

The basic method conformed to a published book ("Antibodies", Harlow, E. et al., (1988), Cold Spring Harbor Lab. Press, New York). That is, the eluted pfotein of Example 6 (3) considered to be α4·IgG heavy chain-β1·IgG heavy chain chimeric protein heterodimer complex was ¹²⁵I-labeled using the lactoperoxidase method. Then, Affigel-10 (Bio-rad) was washed with 0.1 M Hepes solution (pH 8.0), and normal murine IgG, anti-human integrin α4 antibody (clone 11C2B) and anti-human integrin β1 antibody (clone 4B4) were added. Reaction was effected at 4°C for 16 hours to cause covalent bonding, to prepare normal murine IgG beads and the respective antibody beads. Then, the ¹²⁵I labeled α4·IgG heavy chain-β1·IgG heavy chain chimeric protein heterodimer complex and normal murine IgG beads were inverted and mixed at 4 °C for 4 hours for preclearing, and the mixture and the antibody beads were inverted and mixed at 4°C for 16 hours. After completion of mixing, the beads were washed with a washing buffer (200 mM Tris-HCl, 0.5 M NaCl, 0.1% NP-40, 1 mM MgCl₂ or 10 mM EDTA, pH 8.0) three times. After completion of washing, a sample buffer for electrophoresis was added to the beads for treatment at 100 °C for 5 minutes, and the mixture was centrifuged. The supernatant solution was analysed by electrophoresis under reducing condition. After completion of electrophoresis, the gel was dried by a gel dryer, and the protein was detected by autoradiography.

As a result of immunoprecipitation in the presence of 1 mM MgCl₂, from the beads of both the anti-human integrin α4 antibody and the anti-human integrin β1 antibody, the same precipitation patterns expected from the structure of α4·IgG heavy chain-β1·IgG heavy chain chimeric protein heterodimer complex were obtained. Thus, the protein obtained in (3) of Example 6 was identified as α4·IgG heavy chain-β1·IgG heavy chain chimeric protein heterodimer complex.

On the other hand, the immunoprecipitation pattern obtained by using the anti-integrin β1 antibody beads in the presence of 10 mM EDTA was the same as that in the presence of 1 mM MgCl₂, to clarify that the association between integrin α4·IgG heavy chain chimeric protein and integrin β1·IgG heavy chain chimeric protein does not depend on cations. The above results suggest that the eluted protein obtained in (3) of Example 6 was certain α4·IgG heavy chain-β1·IgG heavy chain chimeric protein heterodimer complex, and if the result of (4) of Example 6 is also taken into account, it is strongly suggested that the association between both the proteins is stable association through a disulfide bond existing the IgG heavy chains.

### (2) Examination on the structural stability of α4·IgG heavy chain-β1·IgG heavy chain chimeric protein heterodimer complex by sequential immunoprecipitation

According to (1), ¹²⁵I labeled α4·IgG heavy chain-β1·IgG heavy chain chimeric protein heterodimer complex was caused to react with normal murine IgG beads, anti-human integrin α4 antibody (11C2B) beads or anti-human integrin β1 antibody (4B4) beads at 4°C for 4 hours, and the reaction mixture was washed. After washing, the reaction mixture was boiled at 100°C for 5 minutes in the presence of 2% SDS and centrifuged, and the supernatant (primary immunoprecipitation sample) was diluted to 10 times by 1% BSA-containing PBS, and was again reacted with the anti-integrin β1 antibody beads and the anti-integrin α4 antibody beads at 4°C for 16 hours. After completion of reaction, the beads were washed, and a sample buffer for electrophoresis was added. The mixture was treated at 100°C for 5 minutes and centrifuged. The supernatant solution (secondary immunoprecipitation sample) was analyzed by SDS-PAGE/autoradiography.

As a result, the electrophoretic pattern obtained by the primary immunoprecipitation was also similarly observed in the secondary immunoprecipitation. This result suggests that the association between the α4·IgG heavy chain chimeric protein and the β1·IgG heavy chain chimeric protein in the α4·IgG heavy chain-β1·IgG heavy chain chimeric protein heterodimer complex is not dissociated either by boiling in the presence of 2% SDS, and strongly supports that the complex has a stable heterodimer structure based on a disulfide bond.

### Example 8

### Binding of α4·IgG heavy chain-β1·IgG heavy chain chimeric protein heterodimer complex to VCAM-1

It was examined that the α4·IgG heavy chain-β1·IgG heavy chain chimeric protein heterodimer complex produced by CHO cells can be bound to the ligand of integrin α4β1 by using the cells expressing VCAM-1. Human normal umbilical intravenous endothelial cells were cultured with IL-1 3U/ml for 16 hours, to prepare VCAM-1 expressing cells. The cells were treated by 1 mM EDTA at 37°C for 15 minutes, for dispersion as single cells. The cells (2 x 10⁵ cells per sample tube) were cultured with the supernatant of the CHO cells producing α4·IgG heavy chain-β1·IgG heavy chain chimeric protein heterodimer complex for 30 minutes in the presence of 1 mM (final concentration) MnCl₂ or 3 mM (final concentration) EDTA. After completion of reaction, the cells were washed twice by centrifugation at 1200 rpm at room temperature for 5 minutes using a buffer for binding assay (24 mM Tris-CHl, 10 mM Hepes, 150 mM NaCl, 1 mM MnCl ₂ or 1 mM EDTA, 1% BSA, 2 mM glucose, pH 7.4). After washing, FITC labeled-anti-human IgG antibody (Cappel) was added, and incubated at room temperature for 20 minutes. The cells were washed by the same buffer, and the chimeric proteins bound to the cells were determined by a flow cytometer (ELITE, Coulter).

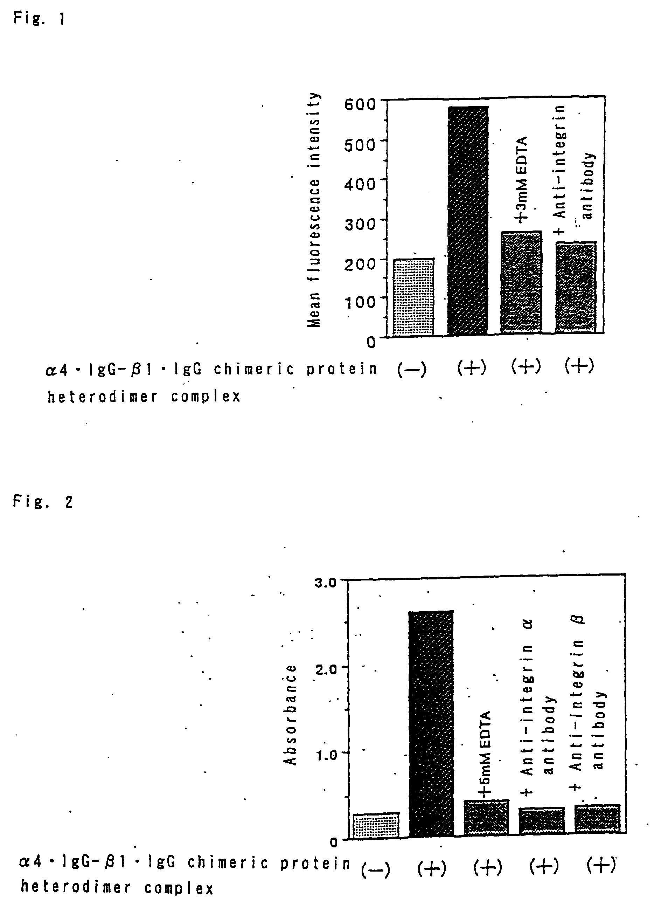

The results are shown in Fig. 1. It was observed that the fluorescence intensity showing the binding of α4·IgG heavy chain-β1·IgG heavy chain chimeric protein heterodimer complex increased by culturing the VCAM-1 expression cells with the supernatant containing α4·IgG heavy chain-β1·IgG heavy chain chimeric protein heterodimer complex. The binding was inhibited by adding anti-human integrin antibodies (anti-α·antibody: clone L25.3, 10 µm/ml + anti-β1 antibody: clone 4B4, 10 µm/ml) or 3 mM EDTA. This result suggests that the α4·IgG heavy chain-β1·IgG heavy chain chimeric protein heterodimer complex can be bound to VCAM-1 like the integrin α4β1 existing on the surfaces of cell membranes, and furthermore that the binding is α4β1-specific and retains a feature of the binding that it is dependent on cations.

### Example 9

### Binding of α4·IgG heavy chain-β1·IgG heavy chain chimeric protein heterodimer complex to a peptide fragment of fibronectin

The capability of α4·IgG heavy chain-β1·IgG heavy chain chimeric protein heterodimer complex to be bound to the peptide fragment (sequence No. 3) of the other ligand, fibronectin was also examined.

At first, according to the said report (Humphries, M. J. et al., J. Biol. Chem., 262, 6886-6892 (1987)), the peptide fragment of sequence No. 3 (CS-1 peptide) was bound to rabbit IgG (Sigma), to prepare CS-1-IgG. The CS-1-IgG was diluted by PBS(-), and put in a 96-well immunoplate (NUNC) by 100 µl/well, and allowed to stand at 4°C for 16 hours, to be formed as a solid phase on the plate.

After completion of standing, the surface of the plate was washed with PBS(-) twice and treated with denatured 1% BSA(heat-natured at 80°C for 10 minutes)-PBS solution ( 300 µl/well) at 4°C for 3 hours to block the nonspecific reaction. Then, the solid phase CS-1-IgG and the CHO culture supernatant (100 µl) containing α4·IgG heavy chain-β1·IgG heavy chain chimeric protein heterodimer complex were reacted with each other at 30°C for 3 hours. The non-bound α4·IgG heavy chain-β1·IgG heavy chain chimeric protein heterodimer complex was removed by washing with 0.1% BSA-containing TBS buffer (150 mM NaCl, 25 mM Tris-HCl, 1 mM MnCl₂, pH 7.4) twice, and the bound α4·IgG heavy chain-β1·IgG heavy chain chimeric protein heterodimer complex was detected by biotin labeled anti-human IgG antibody (Vector) as the primary antibody and avidin labeled horseradish peroxidase (Sigma) as the secondary antibody. The surface of the plate was washed with the TBS buffer. Orthophenylenediamine was added as a substrate to it for color development, and the absorbance at 490 nm were measured.

The results are shown in Fig. 2. The react ion with α4·IgG heavy chain-β1·IgG heavy chain chimeric protein heterodimer complex showed a rise in the absorbance indicating the binding to CS-1 peptide. The binding was almost perfectly inhibited by the presence of anti-integrin αA antibody (clone L25.3), anti-integrin β1 antibody (clone 4B4) or 5 mM EDTA. Therefore, it was clarified that α4·IgG heavy chain-β1·IgG heavy chain chimeric protein heterodimer complex can be bound also to the CS-1 peptide which is a peptide fragment of fibronectin, and that a feature of binding that it depends on cations is retained.

### Example 10

### Evaluation of an inhibitory peptide by using a system for determining the binding of α4·IgG heavy chain-β1·IgG heavy chain chimeric protein heterodimer complex to a peptide fragment of fibronectin

In the binding determination system of Example 9, the effects of three peptides, i.e., sequence No. 16 (hereinafter called GPEILDVPST), 17 (hereinafter called GPEILEVPST) and 18 (hereinafter called GRGDSP) were examined.

Gly Pro Glu Ile Leu Asp Val Pro Ser Thr (Sequence No. 16)
Gly Pro Glu Ile Leu Glu Val Pro Ser Thr (Sequence No. 17)
Gly Arg Gly Asp Ser Pro (Sequence No. 18)

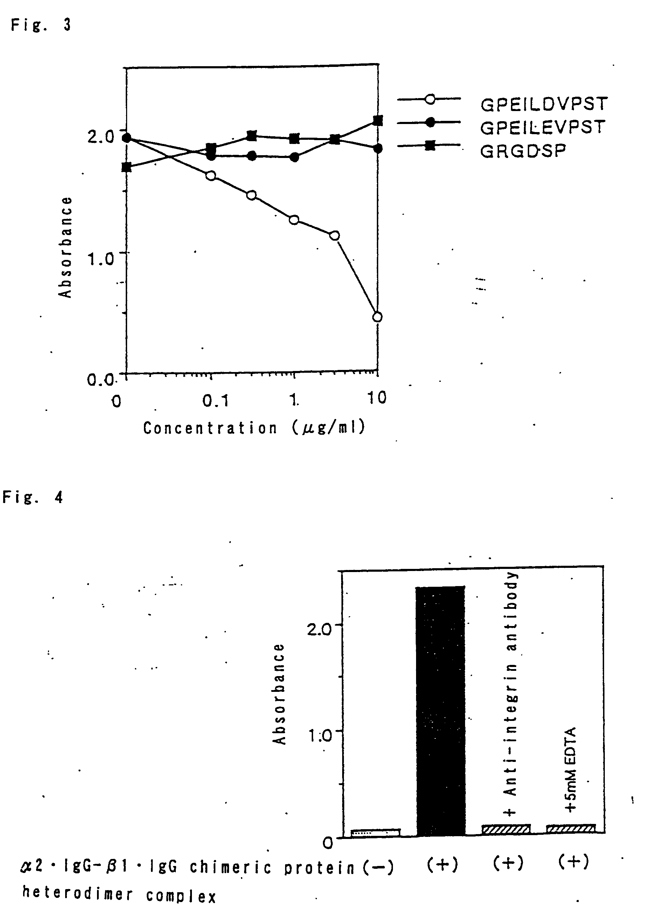

The all peptides were synthesized by a peptide synthesizer. The peptide and 100 µl of CHO cultured supernatant solution containing αα4·IgG heavy chain-β1·IgG heavy chain chimeric protein heterodimer complex were mixed at room temperature for 20 minutes, and the binding to CS-1-IgG was determined according to the method in Example 9. The results are shown in Fig. 3. GPEILDVPST showed temperature-dependent inhibitory activity in a range of 0.1 to 10 µmg/ml, but GPEILEVPST and GRGDSP did not show any inhibition of the binding. These results show that the binding determination system in Example 9 allows to detect the inhibiting effect of the peptide (GPEILDVPST) inhibiting the binding between integrin α4β1 and CS-1 peptide specifically .

### Example 11

### Construction of integrin α2·IgG heavy chain chimeric protein expression vector

The DNA fragment coding for the extracellular portion of integrin α2 was divided into α2-1 and α2-2 based on the reported cDNA sequence information (Takada, Y. et al., J. Cell. Biol., 109, 397-407 (1989)) and subcloned, and they were integrated on an expression vector. At first, the RNA of human fibroblast cell line MRC-5 (ATCC CCL 171) as integrin α2 expressing cell was separated, and an oligo dT cellulose column was used to purify PolyA(+)RNA. Based on it, a single stranged cDNA was synthesized and used as the template of PCR. As PCR primers, DNA oligomers of sequence Nos. 20 and 21 were synthesized for α2-1, and DNA oligomers of sequence Nos. 22 and 23, for α2-2.

5' -GCTCGAGCAAACCCAGCGCAACTACGG-3' (Sequence No. 20)
5' -ATAGTGCCCTGATGACCATTG-3' (Sequence No. 21)
5' -GATGGCTTTAATGATGTGATTG-3' (Sequence No. 22)
5' -TGTTGGTACTTCGGCTTTCTC-3' (Sequence No. 23)

The template cDNA, primers, dNTPs and Taq polymerase were mixed in a PCR buffer and PCR was performed 30 cycles by a thermal cycler (reaction conditions: 94°C 1 minute - 60°C 2 minutes - 72°C 3 minutes). The amplified DNA fragment of α2-1 was digsted by restriction enzymes Xho I and EcoR I , and the DNA fragment of α2-2 was blunted at the termini by T4DNA polymerase treatment and digested by restriction enzyme EcoR I. Each fragment was purified. The two purified DNA fragments were caused to react in a phosphating reaction solution (50 mM Tris-HCl, 10 mM MgCl₂, 25 mM DTT, 1 mM ATP, 0.1 U/µl T4 polynucleotide kinase (Takara), pH 8.0) at 37 °C for 1 hour, and the reaction mixture was heat-treated at 68°C for 5 minutes to inactivate the enzyme. Then, IgG₁SRα prepared in Example 1 was digested by restriction enzyme BamH I and caused to react in Klenow reaction solution (66 mM Tris-HCl, 10 mM MgCl ₂, 10 mM DTT, 0.2 mM dNTPs, 0.05 U/ µl Klenow fragment (Takara), pH 7.5) at 37°C for 30 minutes, to blunt the termini, and the reaction mixture was heat-treated at 70°C for 5 minutes to inactivate the enzyme. Furthermore, a large DNA fragment was digested by restriction enzyme Xho I, and pudified. The two (α2-1 and α2-2) DNA fragments phosphated before were inserted into the large DNA fragment, to obtain a plasmid DNA. The obtained base sequence coding for integrin α2·IgG heavy chain chimeric protein is shown in sequence No. 19. This plasmid (integrin α2·IgGSRα) is hereinafter called α2·IgG heavy chain chimeric protein expression vector.

### Example 12

### Transfection of integrin α2·IgG heavy chain chimeric protein expression vector and integrin β1·IgG heavy chain chimeric protein expression vector into animal cells, and their manifestation

The integrin α2·IgG heavy chain chimeric protein expression vector was transfected into the integrin β1·IgG heavy chain chimeric protein producing CHO cells prepared and stabilized in Example 4, according to the lipofectin method described in Example 4. That is, integrin α2·IgGSRα and pSV2neo (BRL) were mixed at 10 : 1, and the mixture was mixed with lipofectin reagent. The mixture was added dropwise to the cells. Eighteen hours after completion of dropwise addition , the mixture was cultured in a first selective medium for 2 days, and the cells were dispersed by trypsin-EDTA treatment. The cells were suspended in a second selective medium, and the suspension was disseminated into a 96-well plate. Resistant cells were selectively cultured for about 10 days. Then, the amount of integrin α2·IgG heavy chain chimeric protein and the amount of integrin β1·IgG heavy chain chimeric protein produced in the culture supernatant were determined according to the ELISA method (described later), and a clone producing almost the same amounts of both the chimeric proteins was picked up. The clone was cloned twice according to the limiting dilution analysis, to be stabilized as a clone capable of producing α2·IgG heavy chain-β1·IgG heavy chain chimeric protein heterodimer complex.

### Example 13

### Determination of the amounts of integrin α2·IgG heavy chain chimeric protein and integrin β1·IgG heavy chain chimeric protein by the ELISA method

Fifty microliter per well of anti-human integrin α2 antibody (Becton & Dickinson, clone P1E6) or anti-human integrin β1 antibody (clone 4B4) (2 µg/ml each) was put into a 96-well immunoplate , and allowed to stand at 4°C for 16 hours. Then, each well was washed with PBS(-) twice, blocked, and the culture supernatant of the CHO cells grown in second selective medium was properly diluted and reacted with the coated-antibody at room temperature for 1 hour. After the reaction, the surface of the plate was washed with T-PBS twice, and caused to react with biotinated anti-human IgG antibody for 1 hour and with avidin-horseradish peroxidase for 1 hour, and the reaction mixture was washed with PBS(-) twice. After completion of reaction, orthophenylenediamine was used as a substrate for color development, and the absorbance values at 490 nm were measured using a microplate reader. A clone showing a high absorbance value was selected.

### Example 14

### Purification of α2·IgG heavy chain-β1·IgG heavy chain chimeric protein heterodimer complex

### (1) Culture of CHO cells and preparation of cultured supernatant solution

The CHO cells highly capable of producing α2·IgG heavy chain-β1·IgG heavy chain chimeric protein heterodimer complex were cultured in an αMEM(-) medium containing 5% FBS (Ultra-low IgG grade) for 1 day, to reach semiconfluent, and they were cultured on an αMEM(-) medium containing 1%FBS (Ultra-low IgG grade) for 3 days. The culture supernatant was collected, and concentrated to 1/10 volume by ultrafiltration. Then, 1M Hepes solution (pH 8.0) was added to achieve a final concentration of 5 mM, to obtain a starting solution for further purification.

### (2) Protein A colum chromatography

The starting solution for further purification was passed through Prosep Guard column, and applied to Prosep A column. After completion of application, it was washed with 10 times the column volume of PBS (-), and in succession, the proteins were eluted at a pH 6 to 3 gradient of 0.1M citrate buffers. The peak fraction eluted at pH 3 was collected, and 1M Tris-HCl solution (pH 8.5) was added by 0.1 volume for neutralization. The mixture was dialyzed against PBS(-).

### (3) Affinity column chromatography

According to a report (Kirchhofer, D. et al., J. Biol. Chem., 265, 615-618 (1990)), a collagen immobilized column with a collagen (Typel, Sigma) coupled to cyanogen-bromide-activated Sepharose (Sigma) was prepared. Then, the starting solution for further purification was equilibrated in a TBS buffer (150 mM NaCl, 50 mM Tris-HCl, 1 mM MgCl₂, 1 mM MnCl₂, pH 7.5), applied to a column, allowed to stand at room temperature for 3 hours, and washed with 10 times the column volume of a washing buffer (150 mM NaCl, 50 mM Tris-HCl, 1 mM MgCl₂, 1 mM MnCl₂, 100 mM octyl glucopyranoside, pH 7.5). After completion of washing, an elution buffer (20 mM EDTA, 150 mM NaCl, 50 mM Tris-HCl, 50 mM octyl glucopyranoside, pH 7.5) was used to elute the protein bound to the column. The eluate was collected and dialyzed against PBS(-).

### (4) SDS-PAGE

The eluted fraction of (3) was subjected to SDS-PAGE using 7.0% acrylamide gel under non-reducing or under reudcing condition, and the gel was stained with Coummassie-blue. As a result, a band considered to be attributable to α2·IgG heavy chain-β1·IgG heavy chain chimeric protein heterodimer complex was observed. Under reducing condition, two bands (185 kDa and 135 kDa) considered to be attributable to integrin α2·IgG heavy chain chimeric protein and integrin β1·IgG heavy chain chimeric protein were observed. These results suggest that the eluted protein has a molecular structure considered to be αα2·IgG heavy chain-β1·IgG heavy chain chimeric protein heterodimer complex, and is linked by a disulfide bond between the IgG heavy chains.

### Example 15

### Identification of α2·IgG heavy chain-β1·IgG heavy chain chimeric protein heterodimer complex, and examination of its structural stability

The eluted protein of (3) of Example 14 was ¹²⁵I-labeled, and subjected to immunoprecipitation using the beads coupled with normal murine IgG, anti-human integrin α2 antibody (clone P1E6) or anti-human integrin β1 antibody (clone 4B4) as described in Example 7, and to SDS-PAGE/autoradiography under reducing condition.

As a result, in both 1 mM MgCl₂ and 10 mM EDTA, from the beads of both anti-human integrin α2 antibody and anti-human integrin β1 antibody, the same precipitation patterns expected from the structure of α2·IgG heavy chain-β1·IgG heavy chain chimeric protein heterodimer complex could be obtained. These results show that the eluted protein obtained in (3) of Example 14 is certainly α2·IgG heavy chain-β1·IgG heavy chain chimeric protein heterodimer complex, and with the results of (4) of Example 14 also taken into account, it is strongly suggested that the association of both the proteins is stable through a disulfide bond existing the IgG heavy chains.

### Example 16

### Examination on the capability of α2·IgG heavy chain-β1·IgG heavy chain chimeric protein heterodimer complex to be bound to a collagen, and its specificity

The capability of α2·IgG heavy chain-β1·IgG heavy chain chimeric protein heterodimer complex to be bound to a collagen which is a ligand of integrin α2β1 was examined.

At first, a collagen (Cell Matrix Type 3 mg/ml) was diluted to 0.1 µg/ml by 0.02M acetic acid solution, and put in an immunoplate by 100 µl/well, being kept at 4°C for 16 hours. Then, the collagen solution was removed by suction, and the plate was washed with PBS(-) twice for neutralization. Heat-denaturated 1% BSA-PBS solution was put in the plate by 300 µl/well for blocking at room temperature for 3 hours. After completion of blocking, it was rinsed With PBS (-) twice, to prepare a collagen coated plate.

The cultured supernatant of CHO (100 µl) containing α2·IgG heavy chain-β1·IgG heavy chain chimeric protein heterodimer complex was reacted at 30°C for 3 hours. After completion of reaction, as described in Example 9, the amount of bound α2·IgG heavy chain-β1·IgG heavy chain chimeric protein heterodimer complex was determined.

As a result, as shown in Fig. 4, the absorbance showing the binding of α2·IgG heavy chain-β1·IgG heavy chain chimeric protein heterodimer complex to the collagen was increased. The binding was almost perfectly inhibited in the coexistence of 10 µg/ml of anti-human integrin α2 antibody (clone P1E6) and anti-human integrin β1 antibody (clone 4B4), or in the presence of 5 mM EDTA respectively. This result shows that α2·IgG heavy chain-β1·IgG heavy chain chimeric protein heterodimer complex can be bound to a collagen like the integrin α2β1 existing on the surfaces of cell membranes, and furthermore that the binding is α2β1-specific and that the feature of the binding that it depends on cations is retained.

### Example 17

### Acquisition of a peptide capable of being bound to α4·IgG heavy chain-β1·IgG heavy chain chimeric protein heterodimer complex, and evaluation of its inhibitory activity

At first, α4·IgG heavy chain-β1·IgG heavy chain chimeric protein heterodimer complex purified in Example 6, or human IgG was prepared at a proper concentration by PBS (-) and was coated on a plastic plate at 4°C for 16 hours, being formed as a solid phase on a plastic plate. Then, according to a report (Cott, J. K. and Smoth, G. P., Science, 249, 386-390 (1990)), a phage peptide library in which a random six amino acid residues were cyclyzed by the disulfide bond of cysteine at both the ends was prepared and suspended in 0.1% BSA-containing TBS buffer. The phage peptide library was reacted with human IgG at 30°C for 3 hours, to absorb phage peptides capable of being bound to IgG. Then, the non-absorbed phases were reacted with α4·IgG heavy chain-β1·IgG heavy chain chimeric protein heterodimer complex at 30°C for 3 hours, and the reaction mixture was washed with 0.1% BSA-containing TBS buffer twice to remove the phage peptides incapable of being bound to the heterodimer complex. Only the phage peptides capable of being bound were collected after elution with 0.1M glycine-hydrochloric acid (pH 2.2). After collection, the phage was amplified and the above mentioned binding operation was repeated further twice. The only the phage peptides capable of being bound to the heterodimer complex were selectively concentrated. In the final elution operation, phage peptides capable of being bound to the heterodimer complex were eluted using 10 mM EDTA and 0.1M glycine-hydrochloric acid in two steps, and the amino acid sequences of the respective peptides were analyzed. Of them, eight sequences (sequence Nos. 24 to 31) are shown in Table 1. Furthermore, they were examined using the binding assay system of Example 9, and the IC50 values of the four peptide sequences showing binding inhibitory activity are shown in Table 1.

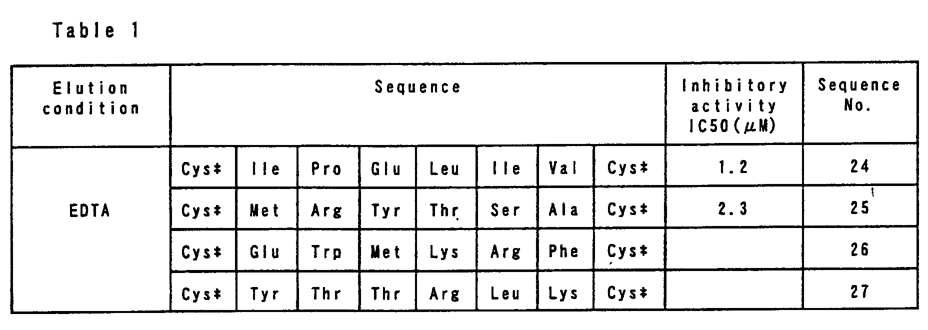

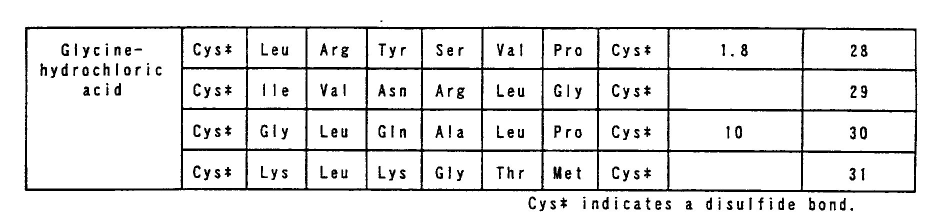

### Example 18

### Acquisition of a low weight molecular compound capable of inhibiting the binding between the peptide fragment on fibronectin and α4·IgG heavy chain-β1·IgG heavy chain chimeric protein heterodimer complex

Reagents and reported compounds were picked up at random, adjusted to a final concentration of 50 or 100 µg/ml, and added to the binding determination system in Example 9. Compounds showing inhibitory activity were obtained. Of the obtained compounds, the binding inhibitory activities of the four compounds of Norethynodrel (Sigma), D-Penicillamine (Aldrich, Weigert, W. M. et al., Angew. Chem. Int. Ed. Eng., 14, 330-336 (1975), γ-2-Naphthyl butyric acid (Fieser, L. F. J. Am. Chem. Soc., 70, 3197-3203 (1948)), 1-Adamantaneacetic acid (Aldrich) were shown in Table 2.

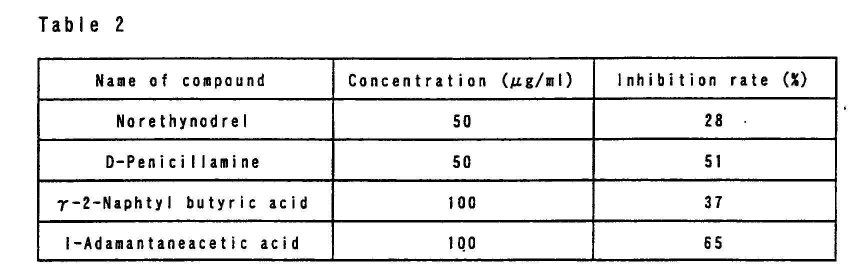

### Example 19

### Preparation of α2·IgG heavy chain-β1·IgG heavy chain chimeric protein heterodimer complex liposome

A liposome was prepared according to the Martin et al.'s method (Martin, F. J. et al., Biochemistry, 20, 4229, (1981)). At first, an activated SH group was introduced into dipalmitoyl phosphatidyl ethanolamine (DPPE, Sigma) using di-crosslinking reagent N-succineimidyl 3-(2-pyridyldithio)propionate (SDPD, Sigma), to prepare pyridylthiopropionyl dipalmitoyl phosphatidyl ethanolamine (PDP-DPPE). The PDP-DPPE, dipalmitoyl phosphatidyl choline (DPPC) and cholesterol were mixed, to prepare a lipid film, and it was treated by a sonicator. Then, a filter was used to obtain a liposome uniform in diameter(PDP-DPPE liposome). Then, α2·IgG heavy chain-β1·IgG heavy chain chimeric protein heterodimer complex or human IgG (Cappel) used as a negative control were dissolved in a Hepes buffer (100 mM Hepes, 150 mM NaCl, pH 8.0), and SDPD was added for reaction for 30 minutes. The reaction solution was applied to PD-10 column (Pharmacia), and eluted by 0.1M acetic acid-sodium acetate buffer (pH 5.5). To the eluate, dithiothreitol was added for treatment for 20 minutes, and the mixture was applied to PD-10 column again and eluted by a Hepes buffer (100 mM Hepes, 150 mM NaCl, pH 8.0), to obtain SDPD coupled α2·IgG heavy chain-β1·IgG heavy chain chimeric protein heterodimer complex. The SDPD modified heterodimer complex and the PDP-DPPE liposome were caused to react with each other at room temperature for 24 hours, and the reaction mixture was separated by Sepharose 4B column (Sigma). From the peak fraction, α2·IgG heavy chain-β1·IgG heavy chain chimeric protein heterodimer complex liposome was obtained.

The amount of α2·IgG heavy chain-β1·IgG heavy chain chimeric protein heterodimer complex bound on the liposome was determined by a densitometer (ATTO) after SDS-PAGE/Coummassie staining, and adjusted to final concentration of 1 mg/ml.

### Example 20

### Flow cytometry of α2·IgG heavy chain-β1·IgG heavy chain chimeric protein heterodimer complex liposome

α2·IgG heavy chain-β1·IgG heavy chain chimeric protein heterodimer complex liposome was dispersed in 1 mM EDTA-containing PBS(-), and caused to react with anti-human integrin α2 antibody (clone P1E6) or anti-human integrin β1 antibody (clone 4B4) at room temperature for 30 minutes. After completion of reaction, the reaction mixture was centrifuged at 15000 rpm for 10 minutes, being followed by washing with 1 mM EDTA-containing PBS(-) and suspended into the solution again. Into the suspension, FITC labeled anti-murine IgG antibody (Cappel, 10 µg/ml) was added as a secondary antibody, and reacted at room temperature for 30 minutes. After completion of reaction, the reaction mixture was similarly washed by centrifugation, and flow cytometry analysis (ELITE, Coulter) was performed.

As a result, the positive reactions for both the antibodies were detected, confirming that α2·IgG heavy chain-β1·IgG heavy chain chimeric protein heterodimer complex was bound on the liposome.

### Example 21

### Binding activity of α2·IgG heavy chain-β1·IgG heavy chain chimeric protein heterodimer complex to a collagen

A collagen (Cell Matrix Type, 3 mg/ml) was diluted by 0.02M acetic acid solution, and the solution was put in an immunoplate by 100 µl/well, being kept at 4°C for 16 hours. Then, the collagen solution was removed by suction, and the plate was washed with PBS(-) twice for neutralization, and heat-denaturated 1% BSA-PBS solution was put in the plate by 300 µl/well for blocking at room temperature for 3 hours. After completion of blocking, the plate was rinsed with PBS(-) twice, to prepare a collagen coated plate.

Normal human plasma (George King) and von Willebrand's factor deficient (severe) plasma (George Kind) were treated with anti-human IgG antibody and protein A, and dialyzed against PBS(-) for 24 hours, to remove the contained sodium citrate. In order that the Ca ion and Mg ion concentration might be a physiological concentration in the blood when used, CaCl₂ and MgCl₂ were added to achieve final concentrations of 1.2 mM and 0.2 mM respectively. Into the normal human plasma and von Willebrand's factor deficient plasma adjusted in cation concentration, α2·IgG heavy chain-β1·IgG heavy chain chimeric protein heterodimer complex liposome or human IgG liposome was suspended to achieve protein concentrations of 1 to 100 ng/ml. Any of the suspensions was put in the collagen coated plate by 100 µl/well. The plate was shaken by a plate shaker at 100 rpm, for react ion at room temperature for 15 minutes. After completion of reaction, the non-bound liposome was removed by washing with a PB solution (1.2 mM CaCl₂, 0.2 mM MgCl₂, 1% BSA-containing PBS, pH 7.4), and the bound liposome was immobilized by 1% glutaraldehyde-PBS at room temperature for 30 minutes. After completion of immobilization, a heat-denaturated BSA-PBS solution was used for blocking at room temperature for 1 hour. Then, as described in Example 16, it was caused to react with biotin labeled human IgG antibody used as a primary antibody and avidin labeled horseradish peroxidase used as a secondary antibody, and washed with a TBS buffer. Into it, orthophenylenediamine was added as a substrate for color development, and the absorbance at 490 nm were measured. To examine the effect of 5 mM EDTA, anti-integrin α2 antibody (clone P1E6, 10 µg/ml) and anti-integrin β1 antibody(clone 4B4, 10 µg/ml), it was caused to react with the liposome suspension at room temperature for 15 minutes before reaction with the collagen.

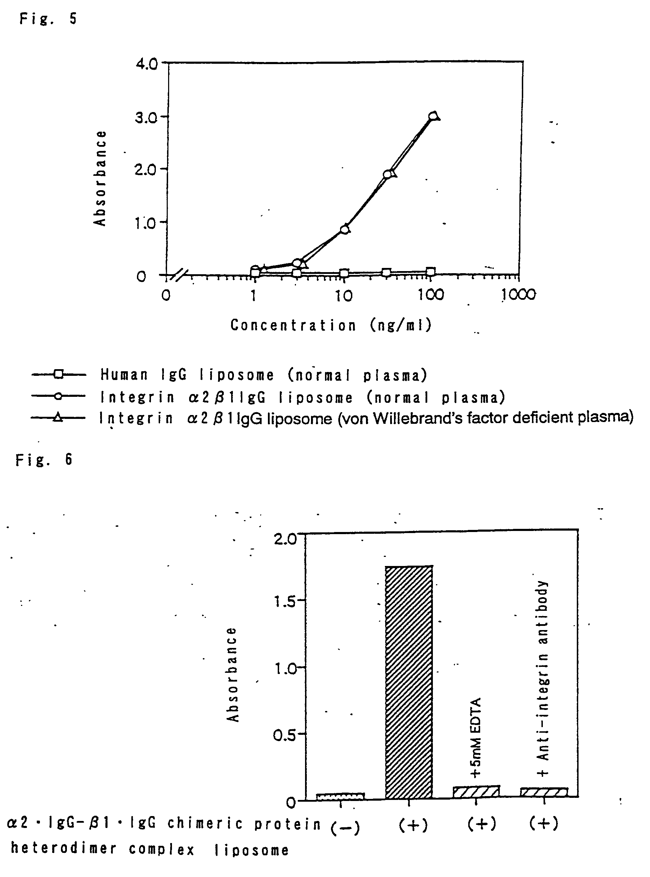

The results are shown in Figs. 5 and 6. In the normal human plasma, the human IgG liposome as a negative control was not found to be bound to the collagen, but the binding of α2·IgG heavy chain-β1·IgG heavy chain chimeric protein heterodimer complex liposome to the collagen was increased with the concentration dependent on manner. Also when the von Willebrand's factor deficient plasma was used, equivalent binding was detected. Furthermore, the binding to the collagen observed when 30 ng/ml of α2·IgG heavy chain-β1·IgG heavy chain chimeric protein heterodimer complex liposome was added to the normal plasma was completely inhibited by adding EDTA as a cation chelating agent or the antibodies. The results show that in plasma with a physiological cation concentrtion, α2·IgG heavy chain-β1·IgG heavy chain chimeric protein heterodimer complex liposome is bound to a collagen like platelets, and strongly suggest that it can be a substitute of adhesive platelets, and can be a reagent for monitoring the collagen exposed region. Furthermore, it is indicated that since equivalent binding activity was shown also in von Willebrand's factor defincient plasma, the liposome can also be used in the plasma with coagulation abnormality such as von Willebrand's disease.

### Example 22

### Analysis of collagen covering state by α2·IgG heavy chain-β1·IgG heavy chain chimeric protein heterodimer complex liposome

Five microliters of a collagen solution was spotted at the center of each of the wells of a Lab-Tek chamber slide (Intermed, 8-well type, plastic) and allowed to stand for 16 hours, then washed and treated for blocking. Then, a suspension in which α2·IgG heavy chain-β1·IgG heavy chain chimeric protein heterodimer complex liposome was suspended in normal human plasma to achieve a protein concentration of 30 ng/ml as described in Example 21 was put in the slide by 200 µl/well, for reaction under the same conditions. After completion of reaction, the non-bound liposome was removed by washing with a PB buffer, and the retained was immobilized and treated for blocking. Then, it was bound to biotin labeled anti-human IgG antibody as a primary antibody and with avidin labeled horseradish peroxidase as a secondary antibody, and was washed with a TBS buffer. After completion of washing, diaminobenzidine was added for staining, to observe the covering state of the α2·IgG heavy chain-β1·IgG heavy chain chimeric protein heterodimer complex liposome bound on the collagen.

With the human IgG liposome, the collagen coated portion was not stained, but with α2·IgG heavy chain-β1·IgG heavy chain chimeric protein heterodimer complex liposome, the collagen coated portion was entirely stained. Therefore, since the α2·IgG heavy chain-β1·IgG heavy chain chimeric protein heterodimer complex liposome covered the collagen coated portion only, it was strongly suggested that the liposome could be a substitute of adhesive platelets.

### Industrial Availability:

The present invention provides integrin-immunoglobulin chimeric protein heterodimer complexes in which the α chain and the β chain of an integrin are stably associated. The obtained integrin-immunoglobulin chimeric protein heterodimer complexes can be directly used as drugs, and can also be used for determining the binding between an integrin and a ligand, and searching for a substance capable of being bound to an integrin and a substance inhibiting the binding between an integrin and a ligand. They can also be used as diagnostic reagents.

Furthermore, among the heterodimer comlexes, especially integrin α2β1-immunoglobulin chimeric protein heterodimer complex can be used as a substitute of platelets. Furthermore, integrin α2β1-immunoglobulin chimeric protein heterodimer complex can be used as a therapeutic or preventive agent for bleeding tendency involved in thrombocytopenia, platelet function abnormality, etc. Furthermore, it can also be used as a reagent for monitoring the exposed region of an extracellular matrix and for the targeting therapy.

Sequence Table

Sequence No. 1
Length of sequence: 4228
Type of sequence: Nucleic acid
Sequence

ATG TTC CCC ACC GAG AGC GCA TGG CTT GGG AAG CGA GGC GCG AAC CCG 48

-35 -30 -25
GGC CCC GAA GCT GCA CTC CGG GAG ACG GTG ATG CTG TTG CTG TGC CTG 96

-20 -15 -10
GGG GTC CCG ACC GGC AGG CCT TAC AAC GTG GAC ACT GAG AGC GCG CTG 144

-5 1 5
CTT TAC CAG GGC CCC CAC AAC ACG CTG TTC GGC TAC TCG GTC GTG CTG 192

10 15 20 25
CAC AGC CAC GGG GCG AAC CGA TGG CTC CTA GTG GGT GCG CCC ACT GCC 240

30 35 40
AAC TGG CTC GCC AAC GCT TCA GTG ATC AAT CCC GGG GCG ATT TAC AGA 288

45 50 55
TGC AGG ATC GGA AAG AAT CCC GGC CAG ACG TGC GAA CAG CTC CAG CTG 336

60 65 70
GGT AGC CCT AAT GGA GAA CCT TGT GGA AAG ACT TGT TTG GAA GAG AGA 384

75 80 85

GAC AAT CAG TGG TTG GGG GTC ACA CTT TCC AGA CAG CCA GGA GAA AAT 432

90 95 100 105
GGA TCC ATC GTG ACT TGT GGG CAT AGA TGG AAA AAT ATA TTT TAC ATA 480

110 115 120
AAG AAT GAA AAT AAG CTC CCC ACT GGT GGT TGC TAT GGA GTG CCC CCT 528

125 130 135
GAT TTA CGA ACA GAA CTG AGT AAA AGA ATA GCT CCG TGT TAT CAA GAT 576

140 145 150
TAT GTG AAA AAA TTT GGA GAA AAT TTT GCA TCA TGT CAA GCT GGA ATA 624

155 160 165
TCC AGT TTT TAC ACA AAG GAT TTA ATT GTG ATG GGG GCC CCA GGA TCA 672

170 175 180 185
TCT TAC TGG ACT GGC TCT CTT TTT GTC TAC AAT ATA ACT ACA AAT AAA 720

190 195 200
TAC AAG GCT TTT TTA GAC AAA CAA AAT CAA GTA AAA TTT GGA AGT TAT 768

205 210 215
TTA GGA TAT TCA GTC GGA GCT GGT CAT TTT CGG AGC CAG CAT ACT ACC 816

220 225 230
GAA GTA GTC GGA GGA GCT CCT CAA CAT GAG CAG ATT GGT AAG GCA TAT 864

235 240 245

ATA TTC AGC ATT GAT GAA AAA GAA CTA AAT ATC TTA CAT GAA ATG AAA 912

250 255 260 265
GGT AAA AAG CTT GGA TCG TAC TTT GGA GCT TCT GTC TGT GCT GTG GAC 960

270 275 280
CTC AAT GCA GAT GGC TTC TCA GAT CTG CTC GTG GGA GCA CCC ATG CAG 1008

285 290 295
AGC ACC ATC AGA GAG GAA GGA AGA GTG TTT GTG TAC ATC AAC TCT GGC 1056

300 305 310
TCG GGA GCA GTA ATG AAT GCA ATG GAA ACA AAC CTC GTT GGA AGT GAC 1104

315 320 325
AAA TAT GCT GCA AGA TTT GGG GAA TCT ATA GTT AAT CTT GGC GAC ATT 1152

330 335 340 345
GAC AAT GAT GGC TTT GAA GAT GTT GCT ATC GGA GCT CCA CAA GAA GAT 1200

350 355 360
GAC TTG CAA GGT GCT ATT TAT ATT TAC AAT GGC CGT GCA GAT GGG ATC 1248

365 370 375
TCG TCA ACC TTC TCA CAG AGA ATT GAA GGA CTT CAG ATC AGC AAA TCG 1296

380 385 390
TTA AGT ATG TTT GGA CAG TCT ATA TCA GGA CAA ATT GAT GCA GAT AAT 1344

395 400 405

AAT GGC TAT GTA GAT GTA GCA GTT GGT GCT TTT CGG TCT GAT TCT GCT 1392

410 415 420 425
GTC TTG CTA AGG ACA AGA CCT GTA GTA ATT GTT GAC GCT TCT TTA AGC 1440

430 435 440
CAC CCT GAG TCA GTA AAT AGA ACG AAA TTT GAC TGT GTT GAA AAT GGA 1488

445 450 455
TGG CCT TCT GTG TGC ATA GAT CTA ACA CTT TGT TTC TCA TAT AAG GGC 1536

460 465 470
AAG GAA GTT CCA GGT TAC ATT GTT TTG TTT TAT AAC ATG AGT TTG GAT 1584

475 480 485
GTG AAC AGA AAG GCA GAG TCT CCA CCA AGA TTC TAT TTC TCT TCT AAT 1632

490 495 500 505
GGA ACT TCT GAC GTG ATT ACA GGA AGC ATA CAG GTG TCC AGC AGA GAA 1680

510 515 520
GCT AAC TGT AGA ACA CAT CAA GCA TTT ATG CGG AAA GAT GTG CGG GAC 1728

525 530 535
ATC CTC ACC CCA ATT CAG ATT GAA GCT GCT TAC CAC CTT GGT CCT CAT 1776

540 545 550
GTC ATC AGT AAA CGA AGT ACA GAG GAA TTC CCA CCA CTT CAG CCA ATT 1824

555 560 565

CTT CAG CAG AAG AAA GAA AAA GAC ATA ATG AAA AAA ACA ATA AAC TTT 1872

570 575 580 585
GCA AGG TTT TGT GCC CAT GAA AAT TGT TCT GCT GAT TTA CAG GTT TCT 1920

590 595 600
GCA AAG ATT GGG TTT TTG AAG CCC CAT GAA AAT AAA ACA TAT CTT GCT 1968

605 610 615
GTT GGG AGT ATG AAG ACA TTG ATG TTG AAT GTG TCC TTG TTT AAT GCT 2016

620 625 630
GGA GAT GAT GCA TAT GAA ACG ACT CTA CAT GTC AAA CTA CCC GTG GGT 2064

635 640 645
CTT TAT TTC ATT AAG ATT TTA GAG CTG GAA GAG AAG CAA ATA AAC TGT 2112

650 655 660 665
GAA GTC ACA GAT AAC TCT GGC GTG GTA CAA CTT GAC TGC AGT ATT GGC 2160

670 675 680
TAT ATA TAT GTA GAT CAT CTC TCA AGG ATA GAT ATT AGC TTT CTC CTG 2208

685 690 695
GAT GTG AGC TCA CTC AGC AGA GCG GAA GAG GAC CTC AGT ATC ACA GTG 2256

700 705 710
CAT GCT ACC TGT GAA AAT GAA GAG GAA ATG GAC AAT CTA AAG CAC AGC 2304

715 720 725

AGA GTG ACT GTA GCA ATA CCT TTA AAA TAT GAG GTT AAG CTG ACT GTT 2352



730 735 740 745

CAT GGG TTT GTA AAC CCA ACT TCA TTT GTG TAT GGA TCA AAT GAT GAA 2400



750 755 760

AAT GAG CCT GAA ACG TGC ATG GTG GAG AAA ATG AAC TTA ACT TTC CAT 2448



765 770 775

GTT ATC AAC ACT GGC AAT AGT ATG GCT CCC AAT GTT AGT GTG GAA ATA 2496



780 785 790

ATG GTA CCA AAT TCT TTT AGC CCC CAA ACT GAT AAG CTG TTC AAC ATT 2588



795 800 805

TTG GAT GTC CAG ACT ACT ACT GGA GAA TGC CAC TTT GAA AAT TAT CAA 2592



810 815 820 825

AGA GTG TGT GCA TTA GAG CAG CAA AAG AGT GCA ATG CAG ACC TTG AAA 2640



830 835 840

GGC ATA GTC CGG TTC TTG TCC AAG ACT GAT AAG AGG CTA TTG TAC TGC 2688



845 850 855

ATA AAA GCT GAT CCA CAT TGT TTA AAT TTC TTG TGT AAT TTT GGG AAA 2736



860 865 870

ATG GAA AGT GGA AAA GAA GCC AGT GTT CAT ATC CAA CTG GAA GGC CGG 2784



875 880 885

CCA TCC ATT TTA GAA ATG GAT GAG ACT TCA GCA CTC AAG TTT GAA ATA 2832

890 895 900 905
AGA GCA ACA GGT TTT CCA GAG CCA AAT CCA AGA GTA ATT GAA CTA AAC 2880

910 915 920
AAG GAT GAG AAT GTT GCG CAT GTT CTA CTG GAA GGA CTA CAT CAT CAA 2928

925 930 935
AGA CCC AAA CGT TAT TTC ACG GAT CCC GAG CTGCTGGAAG CAGGCTCAGC 2978

940 945
GCTCCTGCCT GGACGCATCC CGGCTATGCA GCCCCAGTCC AGGGCAGCAA GGCAGGCCCC 3038
GTCTGCCTCT TCACCCGGAG CCTCTGCCCG CCCCACTCAT GCTCAGGGAG AGGGTCTTCT 3098
GGCTTTTTCC CAGGCTCTGG GCAGGCACAG GCTAGGTGCC CCTAACCCAG GCCCTGCACA 3158
CAAAGGGGCA GGTGCTGGGC TCAGACCTGC CAAGAGCCAT ATCCGGGAGG ACCCTGCCCC 3218
TGACCTAAGC CCACCCCAAA GGCCAAACTC TCCACTCCCT CAGCTCGGAC ACCTTCTCTC 3278
CTCCCAGATT CCAGTAACTC CCAATCTTCT CTCTGCA GAG CCC AAA TCT TGT GAC 3333

950
AAA ACT CAC ACA TGC CCA CCG TGC CCA GGTAAGCCAG CCCAGGCCTC 3380

955 960
GCCCTCCAGC TCAAGGCGGG ACAGGTGCCC TAGAGTAGCC TGCATCCAGG GACAGGCCCC 3440
AGCCGGGTGC TGACACGTCC ACCTCCATCT CTTCCTCA GCA CCT GAA CTC CTG 3493

965
GGG GGA CCG TCA GTC TTC CTC TTC CCC CCA AAA CCC AAG GAC ACC CTC 3541

970 975 980

ATG ATC TCC CGG ACC CCT GAG GTC ACA TGC GTG GTG GTG GAC GTG AGC 3589

985 990 995
CAC GAA GAC CCT GAG GTC AAG TTC AAC TGG TAC GTG GAC GGC GTG GAG 3637

1000 1005 1010 1015
GTG CAT AAT GCC AAG ACA AAG CCG CGG GAG GAG CAG TAC AAC AGC ACG 3685

1020 1025 1030
TAC CGG GTG GTC AGC GTC CTC ACC GTC CTG CAC CAG GAC TGG CTG AAT 3733

1035 1040 1045
GGC AAG GAG TAC AAG TGC AAG GTC TCC AAC AAA GCC CTC CCA GCC CCC 3781

1050 1055 1060
ATC GAG AAA ACC ATC TCC AAA GCC AAA GGTGGGACCC GTGGGGTGCG 3828

1065 1070
AGGGCCACAT GGACAGAGGC CGGCTCGGCC CACCCTCTGC CCTGAGAGTG ACCGCTGTAC 3888
CAACCTCTGT CCTACA GGG CAG CCC CGA GAA CCA CAG GTG TAC ACC CTG 3937

1075 1080
CCC CCA TCC CGG GAT GAG CTG ACC AAG AAC CAG GTC AGC CTG ACC TGC 3985

1085 1090 1095
CTG GTC AAA GGC TTC TAT CCC AGC GAC ATC GCC GTG GAG TGG GAG AGC 4033

1100 1105 1110 1115

AAT GGG CAG CCG GAG AAC AAC TAC AAG ACC ACG CCT CCC GTG CTG GAT 4081

1120 1125 1130
TCC GAC GGC TCC TTC TTC CTC TAC AGC AAG CTC ACC GTG GAC AAG AGC 4129

1135 1140 1145
AGG TGG CAG CAG GGG AAC GTC TTC TCA TGC TCC GTG ATG CAT GAG GCT 4177

1150 1155 1160
CTG CAC AAC CAC TAC ACG CAG AAG AGC CTC TCC CTG TCT CCG GGT AAA 4225

1165 1170 1175
TGA 4228
Sequence No. 2
Length of sequence: 3463
Type of sequence: Nucleic acid
Sequence
ATG AAT TTA CAA CCA ATT TTC TGG ATT GGA CTG ATC AGT TCA GTT TGC 48

-20 -15 -10 -5
TGT GTG TTT GCT CAA ACA GAT GAA AAT AGA TGT TTA AAA GCA AAT GCC 96

1 5 10
AAA TCA TGT GGA GAA TGT ATA CAA GCA GGG CCA AAT TGT GGG TGG TGC 144

15 20 25
ACA AAT TCA ACA TTT TTA CAG GAA GGA ATG CCT ACT TCT GCA CGA TGT 192

30 35 40

GAT GAT TTA GAA GCC TTA AAA AAG AAG GGT TGC CCT CCA GAT GAC ATA 240

45 50 55 60
GAA AAT CCC AGA GGC TCC AAA GAT ATA AAG AAA AAT AAA AAT GTA ACC 288

65 70 75
AAC CGT AGC AAA GGA ACA GCA GAG AAG CTC AAG CCA GAG GAT ATT CAT 336

80 85 90
CAG ATC CAA CCA CAG CAG TTG GTT TTG CGA TTA AGA TCA GGG GAG CCA 384

95 100 105
CAG ACA TTT ACA TTA AAA TTC AAG AGA GCT GAA GAC TAT CCC ATT GAC 432

110 115 120
CTC TAC TAC CTT ATG GAC CTG TCT TAT TCA ATG AAA GAC GAT TTG GAG 480

125 130 135 140
AAT GTA AAA AGT CTT GGA ACA GAT CTG ATG AAT GAA ATG AGG AGG ATT 528

145 150 155
ACT TCG GAC TTC AGA ATT GGA TTT GGC TCA TTT GTG GAA AAG ACT GTG 576

160 165 170
ATG CCT TAC ATT AGC ACA ACA CCA GCT AAG CTC AGG AAC CCT TGC ACA 624

175 180 185
AGT GAA CAG AAC TGC ACC ACC CCA TTT AGC TAC AAA AAT GTG CTC AGT 672

190 195 200

CTT ACT AAT AAA GGA GAA GTA TTT AAT GAA CTT GTT GGA AAA CAG CGC 720

205 210 215 220
ATA TCT GGA AAT TTG GAT TCT CCA GAA GGT GGT TTC GAT GCC ATC ATG 768

225 230 235
CAA GTT GCA GTT TGT GGA TCA CTG ATT GGC TGG AGG AAT GTT ACA CGG 816

240 245 250
CTG CTG GTG TTT TCC ACA GAT GCC GGG TTT CAC TTT GCT GGA GAT GGG 864

255 260 265
AAA CTT GGT GGC ATT GTT TTA CCA AAT GAT GGA CAA TGT CAC CTG GAA 912

270 275 280
AAT AAT ATG TAC ACA ATG AGC CAT TAT TAT GAT TAT CCT TCT ATT GCT 960

285 290 295 300
CAC CTT GTC CAG AAA CTG AGT GAA AAT AAT ATT CAG ACA ATT TTT GCA 1008

305 310 315
GTT ACT GAA GAA TTT CAG CCT GTT TAC AAG GAG CTG AAA AAC TTG ATC 1056

320 325 330
CCT AAG TCA GCA GTA GGA ACA TTA TCT GCA AAT TCT AGC AAT GTA ATT 1104

335 340 345
CAG TTG ATC ATT GAT GCA TAC AAT TCC CTT TCC TCA GAA GTC ATT TTG 1152

350 355 360

GAA AAC GGC AAA TTG TCA GAA GGA GTA ACA ATA AGT TAC AAA TCT TAC 1200

365 370 375 380
TGC AAG AAC GGG GTG AAT GGA ACA GGG GAA AAT GGA AGA AAA TGT TCC 1248

385 390 395
AAT ATT TCC ATT GGA GAT GAG GTT CAA TTT GAA ATT AGC ATA ACT TCA 1296

400 405 410
AAT AAG TGT CCA AAA AAG GAT TCT GAC AGC TTT AAA ATT AGG CCT CTG 1344

415 420 425
GGC TTT ACG GAG GAA GTA GAG GTT ATT CTT CAG TAC ATC TGT GAA TGT 1392

430 435 440
GAA TGC CAA AGC GAA GGC ATC CCT GAA AGT CCC AAG TGT CAT GAA GGA 1440

445 450 455 460
AAT GGG ACA TTT GAG TGT GGC GCG TGC AGG TGC AAT GAA GGG CGT GTT 1488

465 470 475
GGT AGA CAT TGT GAA TGC AGC ACA GAT GAA GTT AAC AGT GAA GAC ATG 1536

480 485 490
GAT GCT TAC TGC AGG AAA GAA AAC AGT TCA GAA ATC TGC AGT AAC AAT 1584

495 500 505
GGA GAG TGC GTC TGC GGA CAG TGT GTT TGT AGG AAG AGG GAT AAT ACA 1632

510 515 520

AAT GAA ATT TAT TCT GGC AAA TTC TGC GAG TGT GAT AAT TTC AAC TGT 1680

525 530 535 540
GAT AGA TCC AAT GGC TTA ATT TGT GGA GGA AAT GGT GTT TGC AAG TGT 1728

545 550 555
CGT GTG TGT GAG TGC AAC CCC AAC TAC ACT GGC AGT GCA TGT GAC TGT 1776

560 565 570
TCT TTG GAT ACT AGT ACT TGT GAA GCC AGC AAC GGA CAG ATC TGC AAT 1824

575 580 585
GGC CGG GGC ATC TGC GAG TGT GGT GTC TGT AAG TGT ACA GAT CCG AAG 1872

590 595 600
TTT CAA GGG CAA ACG TGT GAG ATG TGT CAG ACC TGC CTT GGT GTC TGT 1920

605 610 615 620
GCT GAG CAT AAA GAA TGT GTT CAG TGC AGA GCC TTC AAT AAA GGA GAA 1968

625 630 635
AAG AAA GAC ACA TGC ACA CAG GAA TGT TCC TAT TTT AAC ATT ACC AAG 2016

640 645 650
GTA GAA AGT CGG GAC AAA TTA CCC CAG CCG GTC CAA CCT GAT CCT GTG 2064

655 660 665
TCC CAT TGT AAG GAG AAG GAT GTT GAC GAC TGT TGG TTC TAT TTT ACG 2112

670 675 680

TAT TCA GTG AAT GGG AAC AAC GAG GTC ATG GTT CAT GTT GTG GAG AAT 2160

685 690 695 700
CCA GAG TGT CCC ACT GGT CCA GAG GAT CCC GAG CTGCTGGAAG CAGGCTCAGC 2213

705 710
GCTCCTGCCT GGACGCATCC CGGCTATGCA GCCCCAGTCC AGGGCAGCAA GGCAGGCCCC 2273
GTCTGCCTCT TCACCCGGAG CCTCTGCCCG CCCCACTCAT GCTCAGGGAG AGGGTCTTCT 2333
GGCTTTTTCC CAGGCTCTGG GCAGGCACAG GCTAGGTGCC CCTAACCCAG GCCCTGCACA 2393
CAAAGGGGCA GGTGCTGGGC TCAGACCTGC CAAGAGCCAT ATCCGGGAGG ACCCTGCCCC 2453
TGACCTAAGC CCACCCCAAA GGCCAAACTC TCCACTCCCT CAGCTCGGAC ACCTTCTCTC 2513
CTCCCAGATT CCAGTAACTC CCAATCTTCT CTCTGCA GAG CCC AAA TCT TGT GAC 2568

715
AAA ACT CAC ACA TGC CCA CCG TGC CCA GGTAAGCCAG CCCAGGCCTC 2615

720 725
GCCCTCCAGC TCAAGGCGGG ACAGGTGCCC TAGAGTAGCC TGCATCCAGG GACAGGCCCC 2675
AGCCGGGTGC TGACACGTCC ACCTCCATCT CTTCCTCA GCA CCT GAA CTC CTG 2728

730
GGG GGA CCG TCA GTC TTC CTC TTC CCC CCA AAA CCC AAG GAC ACC CTC 2776

735 740 745
ATG ATC TCC CGG ACC CCT GAG GTC ACA TGC GTG GTG GTG GAC GTG AGC 2824

750 755 760
CAC GAA GAC CCT GAG GTC AAG TTC AAC TGG TAC GTG GAC GGC GTG GAG 2872

765 770 775

GTG CAT AAT GCC AAG ACA AAG CCG CGG GAG GAG CAG TAC AAC AGC ACG 2920

780 785 790 795
TAC CGG GTG GTC AGC GTC CTC ACC GTC CTG CAC CAG GAC TGG CTG AAT 2968

800 805 810
GGC AAG GAG TAC AAG TGC AAG GTC TCC AAC AAA GCC CTC CCA GCC CCC 3016

815 820 825
ATC GAG AAA ACC ATC TCC AAA GCC AAA GGTGGGACCC GTGGGGTGCG 3063

830 835
AGGGCCACAT GGACAGAGGC CGGCTCGGCC CACCCTCTGC CCTGAGAGTG ACCGCTGTAC 3123
CAACCTCTGT CCTACA GGG CAG CCC CGA GAA CCA CAG GTG TAC ACC CTG 3172

840 845
CCC CCA TCC CGG GAT GAG CTG ACC AAG AAC CAG GTC AGC CTG ACC TGC 3220

850 855 860
CTG GTC AAA GGC TTC TAT CCC AGC GAC ATC GCC GTG GAG TGG GAG AGC 3268

865 870 875
AAT GGG CAG CCG GAG AAC AAC TAC AAG ACC ACG CCT CCC GTG CTG GAT 3316

880 885 890 895
TCC GAC GGC TCC TTC TTC CTC TAC AGC AAG CTC ACC GTG GAC AAG AGC 3364

900 905 910

AGG TGG CAG CAG GGG AAC GTC TTC TCA TGC TCC GTG ATG CAT GAG GCT 3412

915 920 925
CTG CAC AAC CAC TAC ACG CAG AAG AGC CTC TCC CTG TCT CCG GGT AAA 3460

930 935 940
TGA 3463
Sequence No. 3
Length of sequence: 13
Type of sequence: Amino acid
Topology: Linear
Kind of sequence: Peptide
Sequence
Cys Leu His Gly Pro Glu Ile Leu Asp Val Pro Ser Thr
1               5                   10
Sequence No. 4
Length of sequence: 31
Type of sequence: Nucleic acid
Number of strands: Single
Topology: Linear
Kind of sequence: Other nucleic acid, synthetic DNA
Sequence
GCGGATCCCG AGCTGCTGGA AGCAGGCTCA G 31
Sequence No. 5
Length of sequence: 27
Type of sequence: Nucleic acid
Number of strands: Single

Topology: Linear
Kind of sequence: Other nucleic acid, synthetic DNA
Sequence
CCTCTAGACG GCCGTCGCAC TCATTTA 27

Sequence No. 6
Length of sequence: 73
Type of sequence: Nucleic acid
Number of strands: Single
Topology: Linear
Kind of sequence: Other nucleic acid, synthetic DNA
Sequence
CTAGACCACC ATGTTCCCCA CCGAGAGCGC ATGGCTTGGG AAGCGAGGCG CGAACCCGGG
CCCCGGAGCT GCA 73

Sequence No. 7
Length of sequence: 65
Type of sequence: Nucleic acid
Number of strands: Single
Topology: Linear
Kind of sequence: Other nucleic acid, synthetic DNA
Sequence
GCTTCGGGGC CCGGGTTCGC GCCTCGCTTC CCAAGCCATG CGCTCTCGGT GGGGAACATG
GTGGT 65

Sequence No. 8
Length of sequence: 51
Type of sequence: Nucleic acid
Number of strands: Single
Topology: Linear

Kind of sequence: Other nucleic acid, synthetic DNA
Sequence
CTCCGGGAGA CGGTGATGCT GTTGCTGTGC CTGGGGGTCC CGACCGGCAG G 51
Sequence No. 9
Length of sequence: 55
Type of sequence: Nucleic acid
Number of strands: Single
Topology: Straight chain
Kind of sequence: Other nucleic acid, synthetic DNA
Sequence
CCTGCCGGTC GGGACCCCCA GGCACAGCAA CAGCATCACC GTCTCCCGGA GTCGA 55
Sequence No. 10
Length of sequence: 37
Type of sequence: Nucleic acid
Number of strands: Single
Topology: Linear
Kind of sequence: Other nucleic acid, synthetic DNA
Sequence
CACTGCAGGC AGGCCTTACA ACGTGGACAC TGAGAGC 37
Sequence No. 11
Length of sequence: 22
Type of sequence: Nucleic acid
Number of strands: Single
Topology: Linear
Kind of sequence: Other nucleic acid, synthetic DNA
Sequence
GCAGAAACCT GTAAATCAGC AG 22

Sequence No. 12
Length of sequence: 22
Type of sequence: Nucleic acid
Number of strands: Single
Topology: Linear
Kind of sequence: Other nucleic acid, synthetic DNA
Sequence

GCATTTATGC GGAAAGATGT GC 22

Sequence No. 13
Length of sequence: 29
Type of sequence: Nucleic acid
Number of strands: Single
Topology: Linear
Kind of sequence: Other nucleic acid, synthetic DNA
Sequence
CGGGATCCGT GAAATAACGT TTGGGTCTT 29

Sequence No. 14
Length of sequence: 22
Type of sequence: Nucleic acid
Number of strands: Single
Topology: Linear
Kind of sequence: Other nucleic acid, synthetic DNA
Sequence
GCGGAAAAGA TGAATTTACA AC 22

Sequence No. 15
Length of sequence: 27

Type of sequence: Nucleic acid
Number of strands: Single
Topology: Linear
Kind of sequence: Other nucleic acid, synthetic DNA
Sequence
GTGGGATCCT CTGGACCAGT GGGACAC 27
Sequence No. 16
Length of sequence: 10
Type of sequence: Amino acid
Topology: Linear
Kind of sequence: Peptide
Sequence
Gly Pro Glu Ile Leu Asp Val Pro Ser Thr
1               5                   10
Sequence No. 17
Length of sequence: 10
Type of sequence: Amino acid
Topology: Linear
Kind of sequence: Peptide
Sequence
Gly Pro Glu Ile Leu Glu Val Pro Ser Thr
1               5                   10
Sequence No. 18
Length of sequence: 6
Type of sequence: Amino acid
Topology: Linear
Kind of sequence: Peptide

Sequence

Gly Arg Gly Asp Ser Pro
 1               5

Sequence No. 19
Length of sequence: 4675
Type of sequence: Nucleic acid
Sequence

ATG GGG CCA GAA CGG ACA GGG GCC GCG CCG CTG CCG CTG CTG CTG GTG     48

                -25                 -20                 -15
TTA GCG CTC AGT CAA GGC ATT TTA AAT TGT TGT TTG GCC TAC AAT GTT     96

            -10                 -5                  1
GGT CTC CCA GAA GCA AAA ATA TTT TCC GGT CCT TCA AGT GAA CAG TTT    144

    5                   10                  15
GGG TAT GCA GTG CAG CAG TTT ATA AAT CCA AAA GGC AAC TGG TTA CTG    192

20                  25                  30                  35
GTT GGT TCA CCC TGG AGT GGC TTT CCT GAG AAC CGA ATG GGA GAT GTG    240

                40                  45                  50
TAT AAA TGT CCT GTT GAC CTA TCC ACT GCC ACA TGT GAA AAA CTA AAT    288

            55                  60                  65
TTG CAA ACT TCA ACA AGC ATT CCA AAT GTT ACT GAG ATG AAA ACC AAC    336

        70                  75                  80

ATG AGC CTC GGC TTG ATC CTC ACC AGC AAC ATG GGA ACT GGA GGT TTT 384

                85                  90                  95
CTC ACA TGT GGT CCT CTG TGG GCA CAG CAA TGT GGG AAT CAG TAT TAC 432

100 105 110 115
ACA ACG GGT GTG TGT TCT GAC ATC AGT CCT GAT TTT CAG CTC TCA GCC 480

120 125 130
AGC TTC TCA CCT GCA ACT CAG CCC TGC CCT TCC CTC ATA GAT GTT GTG 528

135 140 145
GTT GTG TGT GAT GAA TCA AAT AGT ATT TAT CCT TGG GAT GCA GTA AAG 576

150 155 160
AAT TTT TTG GAA AAA TTT GTA CAA GGC CTT GAT ATA GGC CCC ACA AAG 624

                165                 170                 175
ACA CAG GTG GGG TTA ATT CAG TAT GCC AAT AAT CCA AGA GTT GTG TTT 672

180 185 190 195
AAC TTG AAC ACA TAT AAA ACC AAA GAA GAA ATG ATT GTA GCA ACA TCC 720

200 205 210
CAG ACA TCC CAA TAT GGT GGG GAC CTC ACA AAC ACA TTC GGA GCA ATT 768

215 220 225
CAA TAT GCA AGA AAA TAT GCC TAT TCA GCA GCT TCT GGT GGG CGA CGA 816

230 235 240

AGT GCT ACG AAA GTA ATG GTA GTT GTA ACT GAC GGT GAA TCA CAT GAT 864

                245                 250                 255
GGT TCA ATG TTG AAA GCT GTG ATT GAT CAA TGC AAC CAT GAC AAT ATA 912

260 265 270 275
CTG AGG TTT GGC ATA GCA GTT CTT GGG TAC TTA AAC AGA AAC GCC CTT 960

280 285 290
GAT ACT AAA AAT TTA ATA AAA GAA ATA AAA GCG ATC GCT AGT ATT CCA 1008

295 300 305
ACA GAA AGA TAC TTT TTC AAT GTG TCT GAT GAA GCA GCT CTA CTA GAA 1056

310 315 320
AAG GCT GGG ACA TTA GGA GAA CAA ATT TTC AGC ATT GAA GGT ACT GTT 1104

                325                 330                 335
CAA GGA GGA GAC AAC TTT CAG ATG GAA ATG TCA CAA GTG GGA TTC AGT 1152

340 345 350 355
GCA GAT TAC TCT TCT CAA AAT GAT ATT CTG ATG CTG GGT GCA GTG GGA 1200

360 365 370
GCT TTT GGC TGG AGT GGG ACC ATT GTC CAG AAG ACA TCT CAT GGC CAT 1248

375 380 385
TTG ATC TTT CCT AAA CAA GCC TTT GAC CAA ATT CTG CAG GAC AGA AAT 1296

390 395 400

CAC AGT TCA TAT TTA GGT TAC TCT GTG GCT GCA ATT TCT ACT GGA GAA 1344

                405                 410                 415
AGC ACT CAC TTT GTT GCT GGT GCT CCT CGG GCA AAT TAT ACC GGC CAG 1392

420 425 430 435
ATA GTG CTA TAT AGT GTG AAT GAG AAT GGC AAT ATC ACG GTT ATT CAG 1440

440 445 450
GCT CAC CGA GGT GAC CAG ATT GGC TCC TAT TTT GGT AGT GTG CTG TGT 1488

455 460 465
TCA GTT GAT GTG GAT AAA GAC ACC ATT ACA GAC GTG CTC TTG GTA GGT 1536

470 475 480
GCA CCA ATG TAC ATG AGT GAC CTA AAG AAA GAG GAA GGA AGA GTC TAC 1584

                485                 490                 495
CTG TTT ACT ATC AAA AAG GGC ATT TTG GGT CAG CAC CAA TTT CTT GAA 1632

500 505 510 515
GGC CCC GAG GGC ATT GAA AAC ACT CGA TTT GGT TCA GCA ATT GCA GCT 1680

520 525 530
CTT TCA GAC ATC AAC ATG GAT GGC TTT AAT GAT GTG ATT GTT GGT TCA 1728

535 540 545
CCA CTA GAA AAT CAG AAT TCT GGA GCT GTA TAC ATT TAC AAT GGT CAT 1776

550 555 560

CAG GGC ACT ATC CGC ACA AAG TAT TCC CAG AAA ATC TTG GGA TCC GAT 1824

                565                 570                 575
GGA GCC TTT AGG AGC CAT CTC CAG TAC TTT GGG AGG TCC TTG GAT GGC 1872

580 585 590 595
TAT GGA GAT TTA AAT GGG GAT TCC ATC ACC GAT GTG TCT ATT GGT GCC 1920

600 605 610
TTT GGA CAA GTG GTT CAA CTC TGG TCA CAA AGT ATT GCT GAT GTA GCT 1968

615 620 625
ATA GAA GCT TCA TTC ACA CCA GAA AAA ATC ACT TTG GTC AAC AAG AAT 2016

630 635 640
GCT CAG ATA ATT CTC AAA CTC TGC TTC AGT GCA AAG TTC AGA CCT ACT 2064

                645                 650                 655
AAG CAA AAC AAT CAA GTG GCC ATT GTA TAT AAC ATC ACA CTT GAT GCA 2112

660 665 670 675
GAT GGA TTT TCA TCC AGA GTA ACC TCC AGG GGG TTA TTT AAA GAA AAC 2160

680 685 690
AAT GAA AGG TGC CTG CAG AAG AAT ATG GTA GTA AAT CAA GCA CAG AGT 2208

695 700 705
TGC CCC GAG CAC ATC ATT TAT ATA CAG GAG CCC TCT GAT GTT GTC AAC 2256

710 715 720

TCT TTG GAT TTG CGT GTG GAC ATC AGT CTG GAA AAC CCT GGC ACT AGC 2304

                725                 730                 735
CCT GCC CTT GAA GCC TAT TCT GAG ACT GCC AAG GTC TTC AGT ATT CCT 2352

740 745 750 755
TTC CAC AAA GAC TGT GGT GAG GAT GGA CTT TGC ATT TCT GAT CTA GTC 2400

760 765 770
CTA GAT GTC CGA CAA ATA CCA GCT GCT CAA GAA CAA CCC TTT ATT GTC 2448

775 780 785
AGC AAC CAA AAC AAA AGG TTA ACA TTT TCA GTA ACA CTG AAA AAT AAA 2496

790 795 800
AGG GAA AGT GCA TAC AAC ACT GGA ATT GTT GTT GAT TTT TCA GAA AAC 2544

                805                 810                 815
TTG TTT TTT GCA TCA TTC TCC CTA CCG GTT GAT GGG ACA GAA GTA ACA 2592

820 825 830 835
TGC CAG GTG GCT GCA TCT CAG AAG TCT GTT GCC TGC GAT GTA GGC TAC 2640

840 845 850
CCT GCT TTA AAG AGA GAA CAA CAG GTG ACT TTT ACT ATT AAC TTT GAC 2688

855 860 865
TTC AAT CTT CAA AAC CTT CAG AAT CAG GCG TCT CTC AGT TTC CAA GCC 2736

870 875 880

TTA AGT GAA AGC CAA GAA GAA AAC AAG GCT GAT AAT TTG GTC AAC CTC    2784

    885                 890                 895
AAA ATT CCT CTC CTG TAT GAT GCT GAA ATT CAC TTA ACA AGA TCT ACC    2832

900                 905                 910                 915
AAC ATA AAT TTT TAT GAA ATC TCT TCG GAT GGG AAT GTT CCT TCA ATC    2880

                920                 925                 930
GTG CAC AGT TTT GAA GAT GTT GGT CCA AAA TTC ATC TTC TCC CTG AAG    2928

            935                 940                 945
GTA ACA ACA GGA AGT GTT CCA GTA AGC ATG GCA ACT GTA ATC ATC CAC    2976

        950                 955                 960
ATC CCT CAG TAT ACC AAA GAA AAG AAC CCA CTG ATG TAC CTA ACT GGG    3024

    965                 970                 975
GTG CAA ACA GAC AAG GCT GGT GAC ATC AGT TGT AAT GCA GAT ATC AAT    3072

980                 985                 990                 995
CCA CTG AAA ATA GGA CAA ACA TCT TCT TCT GTA TCT TTC AAA AGT GAA    3120

                1000                1005                1010
AAT TTC AGG CAC ACC AAA GAA TTG AAC TGC AGA ACT GCT TCC TGT AGT    3168

            1015                1020                1025
AAT GTT ACC TGC TGG TTG AAA GAC GTT CAC ATG AAA GGA GAA TAC TTT    3216

        1030                1035                1040

CTT AAT GTG ACT ACC AGA ATT TGG AAC GGG ACT TTC GCA TCA TCA ACG 3264

                1045                1050                1055
TTC CAG ACA GTA CAG CTA ACG GCA GCT GCA GAA ATC AAC ACC TAT AAC 3312

1060 1065 1070 1075
CCT GAG ATA TAT GTG ATT GAA GAT AAC ACT GTT ACG ATT CCC CTG ATG 3360

1080 1085 1090
ATA ATG AAA CCT GAT GAG AAA GCC GAA GTA CCA ACA GAT CCC GAG 3405

1095 1100 1105
CTGCTGGAAG CAGGCTCAGC GCTCCTGCCT GGACGCATCC CGGCTATGCA GCCCCAGTCC 3465
AGGGCAGCAA GGCAGGCCCC GTCTGCCTCT TCACCCGGAG CCTCTGCCCG CCCCACTCAT 3525
GCTCAGGGAG AGGGTCTTCT GGCTTTTTCC CAGGCTCTGG GCAGGCACAG GCTAGGTGCC 3585
CCTAACCCAG GCCCTGCACA CAAAGGGGCA GGTGCTGGGC TCAGACCTGC CAAGAGCCAT 3645
ATCCGGGAGG ACCCTGCCCC TGACCTAAGC CCACCCCAAA GGCCAAACTC TCCACTCCCT 3705
CAGCTCGGAC ACCTTCTCTC CTCCCAGATT CCAGTAACTC CCAATCTTCT CTCTGCA 3762
GAG CCC AAA TCT TGT GAC AAA ACT CAC ACA TGC CCA CCG TGC CCA 3807

1110 1115 1120
GGTAAGCCAG CCCAGGCCTC GCCCTCCAGC TCAAGGCGGG ACAGGTGCCC TAGAGTAGCC 3867
TGCATCCAGG GACAGGCCCC AGCCGGGTGC TGACACGTCC ACCTCCATCT CTTCCTCA 3925
GCA CCT GAA CTC CTG GGG GGA CCG TCA GTC TTC CTC TTC CCC CCA AAA 3973

1125 1130 1135
CCC AAG GAC ACC CTC ATG ATC TCC CGG ACC CCT GAG GTC ACA TGC GTG 4021

1140 1145 1150

GTG GTG GAC GTG AGC CAC GAA GAC CCT GAG GTC AAG TTC AAC TGG TAC 4069

                1155                1160                1165
GTG GAC GGC GTG GAG GTG CAT AAT GCC AAG ACA AAG CCG CGG GAG GAG 4117

1170 1175 1180 1185
CAG TAC AAC AGC ACG TAC CGG GTG GTC AGC GTC CTC ACC GTC CTG CAC 4165

1190 1195 1200
CAG GAC TGG CTG AAT GGC AAG GAG TAC AAG TGC AAG GTC TCC AAC AAA 4213

1205 1210 1215
GCC CTC CCA GCC CCC ATC GAG AAA ACC ATC TCC AAA GCC AAA 4255

1220 1225 1230
GGTGGGACCC GTGGGGTGCG AGGGCCACAT GGACAGAGGC CGGCTCGGCC CACCCTCTGC 4315
CCTGAGAGTG ACCGCTGTAC CAACCTCTGT CCTACA GGG CAG CCC CGA GAA CCA 4369

1235
CAG GTG TAC ACC CTG CCC CCA TCC CGG GAT GAG CTG ACC AAG AAC CAG 4417

1240 1245 1250
GTC AGC CTG ACC TGC CTG GTC AAA GGC TTC TAT CCC AGC GAC ATC GCC 4465

                1255                1260                1265
GTG GAG TGG GAG AGC AAT GGG CAG CCG GAG AAC AAC TAC AAG ACC ACG 4513

1270 1275 1280 1285

CCT CCC GTG CTG GAT TCC GAC GGC TCC TTC TTC CTC TAC AGC AAG CTC 4561

1290 1295 1300
ACC GTG GAC AAG AGC AGG TGG CAG CAG GGG AAC GTC TTC TCA TGC TCC 4609

1305 1310 1315
GTG ATG CAT GAG GCT CTG CAC AAC CAC TAC ACG CAG AAG AGC CTC TCC 4657

1320 1325 1330
CTG TCT CCG GGT AAA TGA 4675

                1335
Sequence No. 20
Length of sequence: 27
Type of sequence: Nucleic acid
Number of strands: Single
Topology: Linear
Kind of sequence: Other nucleic acid, synthetic DNA
Sequence
GCTCGAGCAA ACCCAGCGCA ACTACGG 27

Sequence No. 21
Length of sequence: 21
Type of sequence: Nucleic acid
Number of strands: Single
Topology: Linear
Kind of sequence: Other nucleic acid, synthetic DNA
Sequence
ATAGTGCCCT GATGACCATT G 21

Sequence No. 22
Length of sequence: 22
Type of sequence: Nucleic acid
Number of strands: Single
Topology: Linear
Kind of sequence: Other nucleic acid, synthetic DNA
Sequence
GATGGCTTTA ATGATGTGAT TG 22
Sequence No. 23
Length of sequence: 21
Type of sequence: Nucleic acid
Number of strands: Single
Topology: Linear
Kind of sequence: Other nucleic acid, synthetic DNA
Sequence
TGTTGGTACT TCGGCTTTCT C 21
Sequence No. 24
Length of sequence: 8
Type of sequence: Amino acid
Topology: Circular
Kind of sequence: Peptide
Sequence
Cys Ile Pro Glu Leu Ile Val Cys
1               5
Sequence No. 25
Length of sequence: 8

Type of sequence: Amino acid
Topology: Circular
Kind of sequence: Peptide
Sequence
Cys Met Arg Tyr Thr Ser Ala Cys
1               5
Sequence No. 26
Length of sequence: 8
Type of sequence: Amino acid
Topology: Circular
Kind of sequence: Peptide
Sequence
Cys Glu Trp Met Lys Arg Phe Cys
1               5
Sequence No. 27
Length of sequence: 8
Type of sequence: Amino acid
Topology: Circular
Kind of sequence: Peptide
Sequence
Cys Tyr Thr Thr Arg Leu Lys Cys
1               5
Sequence No. 28
Length of sequence: 8
Type of sequence: Amino acid
Topology: Circular
Kind of sequence: Peptide

Sequence

Cys Leu Arg Tyr Ser Val Pro Cys
1               5

Sequence No. 29
Length of sequence: 8
Type of sequence: Amino acid
Topology: Circular
Kind of sequence: Peptide
Sequence

Cys Ile Val Asn Arg Leu Gly Cys
1               5

Sequence No. 30
Length of sequence: 8
Type of sequence: Amino acid
Topology: Circular
Kind of sequence: Peptide
Sequence

Cys Gly Leu Gln Ala Leu Pro Cys
1               5

Sequence No. 31
Length of sequence: 8
Type of sequence: Amino acid
Topology: Circular
Kind of sequence: Peptide
Sequence

Cys Lys Leu Lys Gly Thr Met Cys
1               5

## Claims

1. A chimeric protein comprising the α chain or β chain of an integrin and the heavy chain or light chain of an immunoglobulin.

2. A chimeric protein heterodimer complex, characterized in that a chimeric protein stated in claim 1 comprising the α chain of an integrin and the heavy chain or light chain of an immunoglobulin and a chimeric protein stated in claim 1 comprising the β chain of the integrin and the heavy chain or light chain of the immunoglobulin are associated with each other.

3. A chimeric protein heterodimer complex, according to claim 2, wherein the chimeric proteins stated in claim 1 are associated with each other in any of the following combinations (1), (2) and (3):
(1) An α chain·immunoglobulin heavy chain-β chain·immunoglobulin heavy chain chimeric protein heterodimer complex, in which a chimeric protein comprising the α chain of an integrin and the heavy chain of an immunoglobulin and a chimeric protein comprising the β chain of the integrin and the heavy chain of the immunoglobulin are associated with each other.
(2) An α chain·immunoglobulin heavy chain-β chain·immunoglobulin light chain chimeric protein heterodimer complex, in which a chimeric protein comprising the α chain of an integrin and the heavy chain of an immunoglobulin and a chimeric protein comprising the β chain of the integrin and the light chain of the immunoglobulin are associated with each other.
(3) An α chain·immunoglobulin light chain-β chain·immunoglobulin heavy chain chimeric protein heterodimer complex, in which a chimeric protein comprising the α chain of an integrin and the light chain of an immunoglobulin and a chimeric protein comprising of the β chain of the integrin and the heavy chain of the immunoglobulin are associated with each other.

4. A chimeric protein or a chimeric protein heterodimer complex, according to any one of claims 1 through 3, wherein the α chain of an integrin is α1, α2, α3, α4, α5, α6, α7, α8, α9, αv, αL, αM, αX, αIIb or αE.

5. A chimeric protein or a chimeric protein heterodimer complex, according to any one of claims 1 through 3, wherein the β chain of an integrin is β1, β2, β3, β4, β5, β6, β7 or β8.

6. A chimeric protein heterodimer complex, according to claim 2 or 3, wherein the α chain of an integrin is α4 or α2 and the β chain is β1.

7. A chimeric protein or a chimeric protein heterodimer complex, according to any one of claims 1 through 3, wherein the chimeric protein comprising the α4 of an integrin and the heavy chain of an immunoglobulin is identified as the amino acid sequence of sequence No. 1.

8. A chimeric protein or a chimeric protein heterodimer complex, according to any one of claims 1 through 3, wherein the chimeric protein comprising the α2 of an integrin and the heavy chain of an immunoglobulin is identified as the amino acid sequence of sequence No. 19.

9. A chimeric protein or a chimeric protein heterodimer complex, according to any one of claims 1 through 3, wherein the chimeric protein comprising the β1 of an integrin and the heavy chain of an immunoglobulin is identified as the amino acid sequence of sequence No. 2.

10. A DNA coding for a chimeric protein stated in claim 1.

11. A DNA coding for a chimeric protein stated in claim 1, wherein the α chain of an integrin is α1, α2, α3, α4, α5, α6, α7, α8, α9, αv, αL, αM, αX, αIIb or αE.

12. A DNA coding for a chimeric protein stated in claim 1, wherein the β chain of an integrin is β1, β2, β3, β4, β5, β6, β7 or β8.

13. A DNA, according to claim11, which is identified as the nucleotide sequence of sequence No. 1 or 19.

14. A DNA, according to claim 12, which is identified as the nucleotide sequence of sequence No. 2.

15. A recombinant vector, wherein a DNA stated in claim 10 is functionally linked to an expression control sequence.

16. A recombinant vector, wherein a DNA stated in claim 11 is functionally linked to an expression control sequence.

17. A recombinant vector, wherein a DNA stated in claim 12 is functionally linked to an expression control sequence.

18. A recombinant vector, wherein a DNA stated in claim 13 is functionally linked to an expression control sequence.

19. A recombinant vector, wherein the DNA stated in claim 14 is functionally linked to an expression control sequence.

20. An animal cell, comprising being transfected simultaneously by a recombinant vector in which a DNA coding for a chimeric protein comprising the α chain of an integrin and the heavy chain or light chain of an immunoglobulin is functionally linked to an expression control sequence, and a recombinant vector in which a DNA coding for a chimeric protein comprising the β chain of the integrin and the heavy chain or light chain of the immunoglobulin is functionally linked to an expression control sequence.

21. An animal cell, according to claim 20, which is transfected simultaneously by the recombinant vectors stated in claims 16 and 17.

22. An animal cell, according to claim 20, which is transfected simultaneously by the recombinant vectors stated in claims 18 and 19.

23. A method for producing the chimeric protein heterodimer complex stated in claim 2, comprising culturing the animal cell stated in claim 20.

24. A drug, comprising a chimeric protein or chimeric protein heterodimer complex stated in any one of claims 1 through 9.

25. A drug composition, comprising a chimeric protein or chimeric protein heterodimer complex stated in any one of claims 1 through 9.

26. A platelet substitute, comprising an isolated extracellular matrix receptor as an active ingredient.

27. A platelet substitute, according to claim 26, wherein the extracellular matrix receptor is an integrin.

28. A platelet substitute, according to claim 27, wherein the α chain of an integrin is α1, α2, α3, α4, α5, α6, α7, α8, α9, αv, αL, αM, αX, αIIb or αE.

29. A platelet substitute, according to claim 27, wherein the β chain of an integrin is β1, β2, β3, β4, β5, β6, β7 or β8.

30. A platelet substitute, according to claim 27, wherein the integrin is integrin α2β1.

31. A platelet substitute, according to claim 26, wherein the extracellular matrix receptor is a chimeric protein heterodimer complex comprising an extracellular matrix receptor and an immunoglobulin.

32. A platelet substitute, according to claim 31, wherein the chimeric protein heterodimer complex is a chimeric protein heterodimer complex comprising an integrin and an immunoglobulin.

33. A platelet substitute, according to claim 32, wherein the chimeric protein heterodimer complex is the chimeric protein heterodimer complex stated in claim 2.

34. A platelet substitute, according to claim 33, wherein the chimeric protein heterodimer complex is the chimeric protein heterodimer complex stated in claim 6.

35. A platelet substitute, according to any one of claims 26 through 34, wherein the extracellular matrix receptor is bound to a carrier when used.

36. A platelet substitute, according to any one of claims 26 through 35, which is hemostatic.

37. A method for testing the binding between a chimeric protein heterodimer complex stated in any one of claims 2 to 9, and a ligand or cells, comprising the steps of bringing a chimeric protein heterodimer complex comprising an integrin and an immunoglobulin, and a ligand or cells into contact with each other, to prepare a mixture, and measuring the amount of the chimeric protein heterodimer complex bound to the ligand or cells or the amount of the ligand or cells bound to the chimeric protein heterodimer complex.

38. A method for searching for a substance capable of being bound to an integrin, comprising using a chimeric protein heterodimer complex stated in any one of claims 2 though 9.

39. A substance capable of being bound to an integrin, obtained by using the method stated in claim 38.

40. A method for searching for a substance which inhibits the binding between an integrin and a ligand, comprising using the method stated in claim 37.

41. A method, according to claim 40, wherein the ligand is a fibronectin fragment identified as sequence No. 3 or a collagen.

42. A protein, peptide or low molecular weight compound which inhibits the binding between an integrin and a ligand, obtained by using the method stated in claim 40 or 41.

43. A method for measuring the amount of a ligand of an integrin, comprising using a chimeric protein heterodimer complex stated in any one of claims 2 through 9.

44. A method for identifying an extracellular matrix exposed region, comprising using a chimeric protein heterodimer complex stated in any one of claims 2 through 9.
